(19) Europäisches Patentamt / European Patent Office / Office européen des brevets

(11) **EP 4 722 329 A1**

(12) **EUROPEAN PATENT APPLICATION**
published in accordance with Art. 153(4) EPC

(43) Date of publication:
**08.04.2026 Bulletin 2026/15**

(21) Application number: **23939775.5**

(22) Date of filing: **09.11.2023**

(51) International Patent Classification (IPC):
**C12M 1/00** *(2006.01)*

(52) Cooperative Patent Classification (CPC):
**C12M 1/00**

(86) International application number:
**PCT/JP2023/040475**

(87) International publication number:
**WO 2024/247307 (05.12.2024 Gazette 2024/49)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **29.05.2023 JP 2023088197**

(71) Applicants:
• **OMRON Corporation**
  **Kyoto-shi, Kyoto 600-8530 (JP)**
• **CHUGAI SEIYAKU KABUSHIKI KAISHA**
  **Tokyo 115-8543 (JP)**

(72) Inventors:
• **SHIBATA, Yoshiya**
  **Kyoto-shi, Kyoto 600-8530 (JP)**

• **NAKASHIMA, Chisato**
  **Kyoto-shi, Kyoto 600-8530 (JP)**
• **HASHIMOTO, Takuma**
  **Kyoto-shi, Kyoto 600-8530 (JP)**
• **YASE, Satoshi**
  **Kyoto-shi, Kyoto 600-8530 (JP)**
• **KITAJIMA, Hiroshi**
  **Kyoto-shi, Kyoto 600-8530 (JP)**
• **KURISU, Takanori**
  **Kyoto-shi, Kyoto 600-8530 (JP)**
• **KURITA, Masashi**
  **Kyoto-shi, Kyoto 600-8530 (JP)**

(74) Representative: **SONN Patentanwälte GmbH & Co KG**
**Riemergasse 14**
**1010 Wien (AT)**

(54) **CELL CULTURE PLAN CREATION DEVICE AND CELL CULTURE SYSTEM**

(57)     In a cell culture plan creation device (40), an input unit (142) receives input of a cell culture end time and a target cell amount at the culture end time, and a plan creation unit (144) that creates a cell culture plan including a first execution instruction for one or more passage processes sequentially executed so as to obtain the target cell amount at the culture end time, and a second execution instruction for a culture process executed following each passage process. In the cell culture plan, the first execution instruction includes information to identify a type of the passage process and information to identify a timing to execute the passage process.

FIG.2

**EP 4 722 329 A1**

## Description

Technical Field

The present disclosure relates to a cell culture plan creation device and a cell culture system.

Background Art

In order to perform an experiment, such as an assay on target cells, there is a need to culture and prepare cells of an amount needed for the experiment.

In relation to a work plan in the field of cell culturing, there is a proposal for a cell culture system that cultures, separates, and grows-on cells collected from a collection subject to form stem cells, so as to obtain cultured cells or cultured tissue. In such a system, when an expected shipment date for shipping cultured cells or cultured tissue has been set from the date the cultured cell are to be used, each item is automatically computed based on a standard culture schedule for a sequence of items of culture completion time, passage execution time, growth factor introduction time, culture start time, separation work time, acceptance test time, acceptance time, and a schedule is identified for each item (see Patent Document 1).

Related Art

Patent Literature

Patent Document 1: Japanese Patent Application Laid-Open (JP-A) No. 2004-290147.

SUMMARY OF INVENTION

Technical Problem

The system described in Patent Document 1 is a system for creating a work plan directed toward culturing cells collected from a patient, and generating tissue to be transplanted into the patient, and accordingly emphasis is placed on monitoring for abnormal cells, such as those that have become cancerous or the like, and on confirming quality as transplant tissue. There is, however, no reference in Patent Document 1 to post-culture cell amount, and there is no suggestion related to creating a plan for culturing a required amount of cells.

An object of the present disclosure is to create a plan for culturing a required amount of cells in cases in which, for example, a specific amount of cells is required for a follow-on experiment.

Solution to Problem

In order to achieve the above object, a cell culture plan creation device according to the present disclosure includes an input unit that receives input of a cell culture end time and a target cell amount at the culture end time, and includes a plan creation unit that creates a cell culture plan including a first execution instruction for one or more passage processes sequentially executed so as to obtain the target cell amount at the culture end time, and a second execution instruction for a culture process executed following each passage process, wherein, in the cell culture plan, the first execution instruction includes information to identify a type of the passage process and information to identify a timing to execute the passage process.

Moreover, a cell culture system according to the present disclosure includes a cell culture plan creation device including an input unit that receives input of cell culture end time and a target cell amount at the culture end time, and a plan creation unit that creates a cell culture plan including a first execution instruction for one or more passage processes sequentially executed so as to obtain the target cell amount at the culture end time and a second execution instruction for a culture process executed following each passage process. In the cell culture plan, the first execution instruction includes information to identify a type of the passage process and information to identify a timing to execute the passage process, and includes a robot system that includes a mobile manipulator equipped with a mechanism to move over a floor and a mechanism to grip an object and a workbench robot that is fixed to a workbench and is equipped with a mechanism to grip an object on the workbench, and that executes a cell culture experiment according to the cell culture plan.

Advantageous Effects

The cell culture plan creation device and cell culture system according to the present disclosure enable creation of a plan to culture cells of a required amount, for example when a specific amount of cells is needed for a follow-on experiment.

BRIEF DESCRIPTION OF DRAWINGS

Fig. 1 is a block diagram illustrating a hardware configuration of a cell culture planning device.
Fig. 2 is a block diagram illustrating an example of a functional configuration of a cell culture planning device according to a first exemplary embodiment.
Fig. 3 is a diagram illustrating an example of a cell DB;
Fig. 4 is a block diagram illustrating an example of protocol data.
Fig. 5 is a flowchart illustrating a flow of cell culture plan creating processing according to the first exemplary embodiment.
Fig. 6 is a diagram illustrating an example of a schematic cell culture plan of a first case.
Fig. 7 is a diagram illustrating a display example of a cell culture plan of the first case.
Fig. 8 is a diagram illustrating other examples of a second graphic.
Fig. 9 is a diagram illustrating an example of a schematic cell culture plan of a second example.
Fig. 10 is a diagram illustrating a display example of a cell culture plan of the second case;
Fig. 11 is a diagram illustrating an example of a schematic cell culture plan of a third case.
Fig. 12 is a diagram illustrating a display example of a cell culture plan of a third case.
Fig. 13 is a schematic plan view illustrating an example of an experiment environment for running a cell culture system.
Fig. 14 is a block diagram illustrating a schematic configuration of a cell culture system according to a second exemplary embodiment.
Fig. 15 is a perspective view of the external appearance of a mobile manipulator.
Fig. 16 is a perspective view of the external appearance of a workbench robot.
Fig. 17 is a block diagram illustrating an example of a functional configuration of a work instruction device according to the second exemplary embodiment.
Fig. 18 is a diagram illustrating an example of experiment definition data of a thawing process.
Fig. 19 is a diagram illustrating an example of experiment definition data of a maintenance passage process.
Fig. 20 is a diagram illustrating an example of experiment definition data of an expansion passage process.
Fig. 21 is a diagram illustrating an example of follow-on experiment data.
Fig. 22 is a flowchart illustrating a flow of a work instruction processing according to a second exemplary embodiment.
Fig. 23 is a flowchart illustrating a flow of control processing by an integration controller.
Fig. 24 is a flowchart illustrating a flow of a passage process.
Fig. 25 is a diagram to explain seeding of cells in a second container.
Fig. 26 is a block diagram illustrating a schematic configuration of a cell culture system according to a third exemplary embodiment.
Fig. 27 is a block diagram illustrating an example of a functional configuration of a cell culture planning device according to the third exemplary embodiment.
Fig. 28 is a block diagram illustrating an example of a functional configuration of a work instruction device according to the third exemplary embodiment.
Fig. 29 is a flowchart illustrating a flow of a cell culture plan creating processing according to the third exemplary embodiment.
Fig. 30A is a diagram to explain a different configuration of a controller.
Fig. 30B is a diagram to explain a different configuration of a controller.
Fig. 31A is a diagram to explain a different configuration of a controller.
Fig. 31B is a diagram to explain a different configuration of a controller.

DESCRIPTION OF EMBODIMENTS

Description follows regarding examples of exemplary embodiments of the present disclosure, with reference to the drawings. Note that the same reference numerals will be appended across the drawings to the same or equivalent configuration elements and parts. Moreover, dimensions and proportions in the drawings are exaggerated for ease of explanation, and sometimes differ from actual proportions.

First Exemplary Embodiment

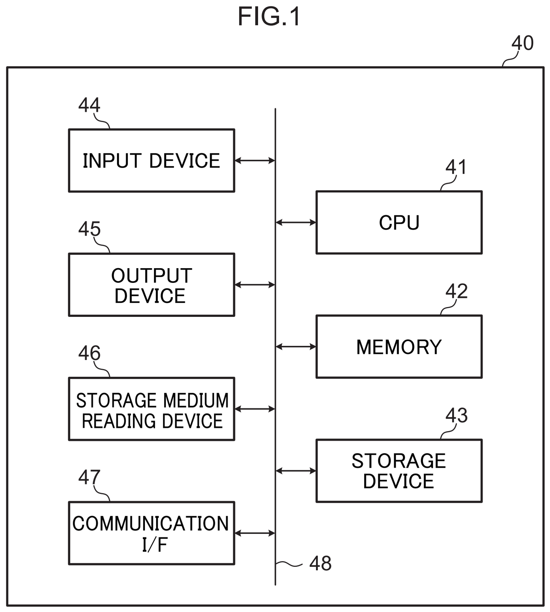

Fig. 1 is a block diagram illustrating a hardware configuration of a cell culture plan creation device 40 according to a first exemplary embodiment. As illustrated in Fig. 1, the cell culture plan creation device 40 includes a central processing unit (CPU) 41, memory 42, a storage device 43, an input device 44, an output device 45, a storage medium reading device 46, and a communication interface (I/F) 47. These configurations are connected together through a bus 48 so as to be capable of communicating with each other.

A program for executing cell culture plan creation processing, described later, is stored in the storage device 43. The CPU 41 is a central processing unit that executes various programs and controls each configuration. Namely, the CPU 41 reads a program from the storage device 43 and executes the program using the memory 42 as a work area. The CPU 41 controls the above configurations and performs various computation processing according to the program stored on the storage device 43.

The memory 42 is configured by random access memory (RAM), and is employed as a work area to temporarily store programs and data. The storage device 43 is configured by read only memory (ROM), and a hard disk drive (HDD), a solid state drive (SSD), or the like, and various programs including an operating system and various data are stored thereon.

The input device 44 is a device for performing various inputs and is, for example, a keyboard, a mouse, or the like. The output device 45 is a device for outputting various information and is, for example, a display, a printer, or the like. The output device 45 may also function as the input device 44 by utilizing a touch panel display therefor.

The storage medium reading device 46 performs reading of data stored on various storage mediums, such as compact disk (CD)-ROM, digital versatile disc (DVD)-ROM, Blu-ray disc, universal serial bus (USB) memory, or the like, and performs writing of data to these storage mediums. The communication I/F 47 is an interface for communication with other devices, and employs a standard such as, for example, Ethernet (registered trademark), FDDI, Wi-Fi (registered trademark), or the like.

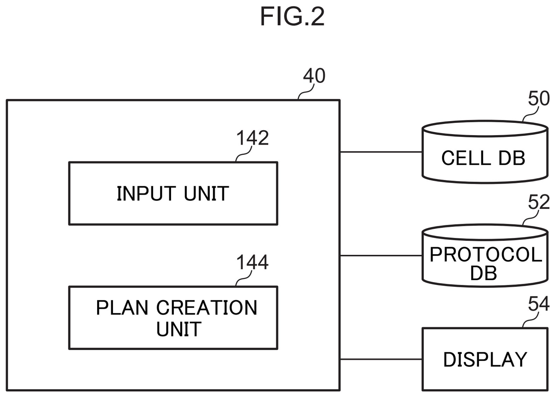

Next, description follows regarding a functional configuration of the cell culture plan creation device 40. Fig. 2 is a block diagram illustrating an example of a functional configuration of the cell culture plan creation device 40. As illustrated in Fig. 2, the cell culture plan creation device 40 is connected to a cell DB 50, a protocol DB 52, and a display 54. The cell DB 50 and the protocol DB 52 may be stored in internal storage areas of the cell culture plan creation device 40, may be stored in a storage device independent to the cell culture plan creation device 40, and may be stored in an external storage device accessible over a network. The display 54 may be a display that is also an example of the input device 44, and may be provided independent to the cell culture plan creation device 40.

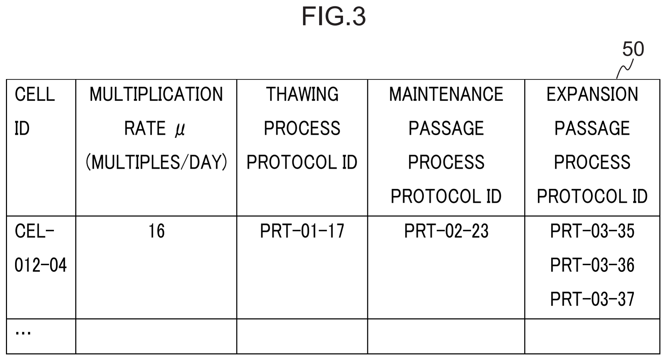

The cell DB 50 is a database stored with information to identify a cell multiplication rate and processing to be applied to cells in a cell culture plan. Fig. 3 illustrates an example of the cell DB 50. In the example of Fig. 3, the cell DB 50 is stored with a cell ID, which is cell identification information, a multiplication rate, and a protocol ID (described in detail later) for each process.

In the example of Fig. 3, the notation format of the cell ID is "CEL-aaa-bb". CEL is a text string indicating that it is a cell ID, aaa is a number representing a type of cell, and bb is a number to discriminate an acquisition route, mutant strain, or the like.

The multiplication rate is information to compute a post-culture cell amount. The cell amount is, for example, a number of cells as expressed by a unitless number or a cell number, a cell concentration (for example, cell number/ml) given a certain amount of the liquid containing cells, a number of containers given a certain number of cells inside a single container, or the like. The cell amount is expressed as "units" in the present exemplary embodiment. The number of cells corresponding to a single unit may be freely defined by a user roughly from the number of cells for seeding when performing a maintenance passage process (described in detail later). For example, a single unit may be defined as being equal to 5000 individual (cell number) or the like.

The protocol ID of each process is identification information of protocol data (described in detail later) to be applied in each process on the cells. In the example of Fig. 3, the protocol ID is stored for each process of a thawing process, a maintenance passage process, and an expansion passage process (described in detail later). In the example of Fig. 3, the notation format of protocol ID is "PRT-kk-ll". PRT is a text string indicating that it is a protocol ID. kk is a number expressing a type of process corresponding to a protocol and, for example, 01 is a thawing process, 02 is a maintenance passage process, 03 is an expansion passage process, and the like. ll is an identification number within the same type of protocol.

As illustrated in Fig. 3, plural protocol IDs may be associated with the same type of processing for cells of the same cell ID. For example, in a first case to a third case of a cell culture plan, described later, the content of expansion passage processes is different in each case for an expansion passage process of cell ID "CEL-012-04". The protocol IDs of at least three types of expansion passage process are stored in the cell DB 50 for this cell ID, resulting in the selection of a different expansion passage process protocol ID for each case therein.

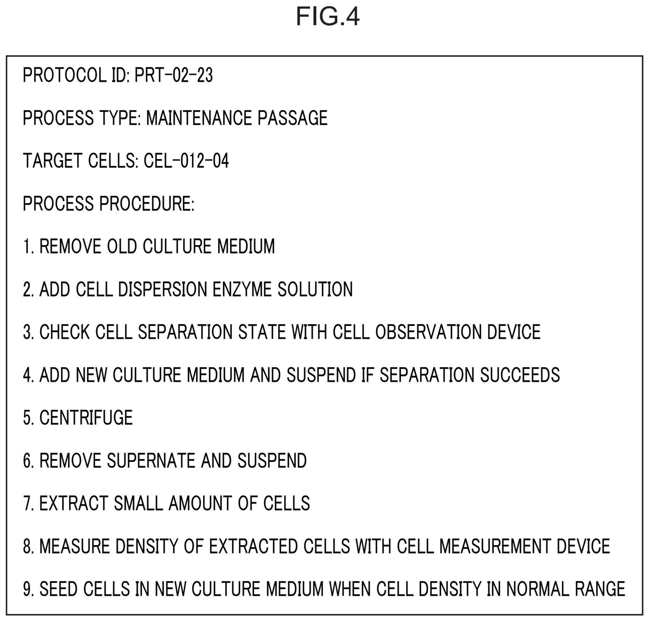

The protocol DB 52 is a database stored with plural protocol data corresponding to the type of various processes contained in the cell culture plan created by the cell culture plan creation device 40. The protocol data is data stored with a protocol ID, a process type, target cells, a processing procedure, and the like in text format, as illustrated in Fig. 4. Note that Fig. 4 is an example of protocol data for a maintenance passage process. Types and amounts of reagent and culture medium, types of container for seeding, and the like are also instructed in actual protocol data. Information about the number of containers for seeding is included when it is protocol data for an expansion passage process by seeding plural containers.

Moreover, as illustrated in Fig. 2, the cell culture plan creation device 40 includes, as functional configuration, an input unit 142, and a plan creation unit 144. Each functional configuration is implemented by the CPU 41 reading a cell culture plan creation program stored on the storage device 43 and expanding and executing the cell culture plan creation program in the memory 42.

The input unit 142 receives input of a cell ID of processing target cells, a cell culture end time, and a target cell amount at culture end time.

The plan creation unit 144 creates a cell culture plan that includes a first execution instruction for one or more passage process sequentially executed so as to obtain the target cell amount at the culture end time, and includes a second execution instruction for a culture process executed following each of the passage processes. The plan creation unit 144 creates a cell culture plan including, as the first execution instruction, information to identify the type of passage process, and information to identify a timing to execute the passage process.

The passage process is processing to move cells to the other container with the objective of taking part of the cells cultured in a container and continuing culturing these cells in a different container. The type of passage process identified in the first execution instruction contains at least one out of a type of passage process belonging to maintenance passage processes, or a type of passage process belonging to expansion passage processes.

Maintenance passage processes are processes in which a small amount of cells appropriate to the objective of continuing to nurture cells by repeated passage processes is extracted from out of the cells that were cultured in the immediately preceding passage process, and these are seeded in a new culture medium. In a maintenance passage process executed from the second time onwards, often about the same number of cells are extracted as the initial cell number in the cell culturing performed immediately preceding that passage process. Expansion passage processes are processes performed for the objective of increasing the usable cell number for an experiment, and are processes in which more cells are extracted from out of cells cultured in the immediately preceding passage process (for example, twice as many or more) than in maintenance passage processes, with these then seeded in a new culture medium. Often plural containers of the same container size as was used for culturing in the immediately preceding passage process are employed as containers for the new culture medium, or a single larger container is employed therefor.

Moreover, a first execution instruction for one or more passage process assumes that the plan creation unit 144 is able to create a cell culture plan including a first execution instruction for plural sequentially executed passage processes. Furthermore, there is an assumption that a cell culture plan including a first execution instruction for a single passage process can be created. For example, sometimes an experiment now needs to be performed suddenly, such as in assay pre-processing on cells nurtured by repeatedly executing unlimited maintenance passage processes, and sometimes a plan is to be created that enables culturing in a shortest possible time period to obtain cells of the required amount by performing an expansion passage process once.

Specifically, the plan creation unit 144 identifies a protocol ID stored in the cell DB 50 as information to identify the type of passage process. In cases in which plural protocol IDs for expansion passage processes are stored in the cell DB 50, the plan creation unit 144 identifies a protocol ID to indicate a protocol of expansion passage processes capable of realizing the target cell amount. When doing so, the plan creation unit 144 references the protocol DB 52 for the content of each protocol.

Moreover, the plan creation unit 144 identifies, as a timing to execute a passage process, a timing to start the passage process, a time limit to end the passage process, and the like. The limit timing to end a passage process is assumed to be slightly before a limit to end actual processing. Moreover, as the timing to execute the passage process, the plan creation unit 144 may indirectly identify a timing to execute the passage process by identifying a timing to end the culture process immediately preceding the passage process, or a timing to start the culture process immediately after the passage process. Note that the representation of time may, as well as a date and time, be a relative period of time to a date and time that serves as a reference (for example, an elapsed time period from the reference date and time).

The plan creation unit 144 may identify in the first execution instruction both a type of passage process belonging to maintenance passage processes, and a type of passage process belonging to expansion passage processes. In such cases, the plan creation unit 144 identifies timings to execute respective passage processes such that a timing to execute the passage process belonging to expansion passage processes is later than the timing to execute the passage process belonging to maintenance passage processes.

Furthermore, the plan creation unit 144 may create a cell culture plan including a third execution instruction for a cell thawing process and for an initial culture process of the thawed cells that are executed in advance of the first most passage process. The third execution instruction includes information to identify the timing to execute the thawing process.

Note that the information to identify the type of thawing process (protocol ID) is possibly made common to many types of cell, and so there might be no need to be instructed individually. However, the third execution instruction for the thawing process may including information to identify the type of thawing process.

Moreover, the plan creation unit 144 outputs, as a signal to display on the display 54, the created cell culture plan with first graphics representing the passage processes and second graphics representing the culture processes arranged along a first direction according to process order in which each of the passage processes and the culture processes is executed. The plan creation unit 144 may employ second graphics that are graphics having a size in a second direction orthogonal to the first direction that increases according to elapse of time in the first direction. In particular, the second graphic facilitates a state of increase of cell amount to be intuitively grasped by making lengths in the second direction at the start time and end time of culturing respectively correspond to the approximate cell amounts at each time.

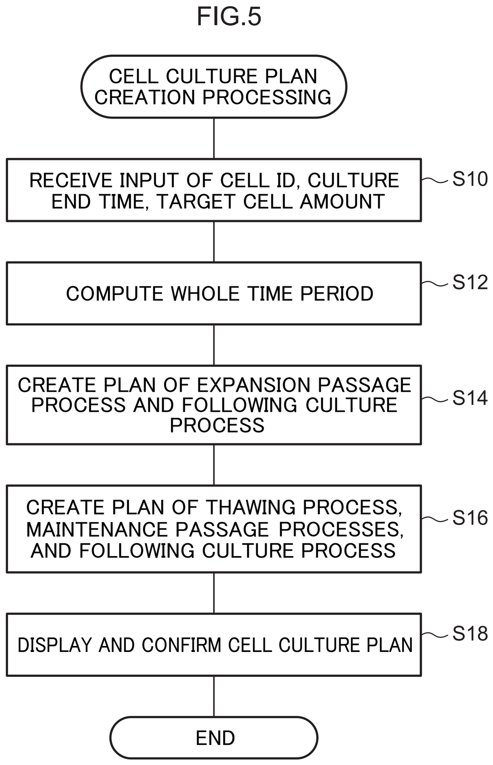

Next, description follows regarding operation of the cell culture plan creation device 40 according to the first exemplary embodiment. Fig. 5 is a flowchart illustrating a flow of cell culture plan creation processing executed by the CPU 41 of the cell culture plan creation device 40.

At step S10, the input unit 142 receives input of a cell ID of cells to be processed, a culture end time, and a target cell amount, and passes these across to the plan creation unit 144.

Next, at step S12, the plan creation unit 144 computes the whole time period of the culture plan from a difference between the culture end time and the culture start time along line. The culture start time may, for example, be a current time, a separately instructed process start time, or the like.

Next, at step S14, the plan creation unit 144 creates a plan for an expansion passage process and a culture process subsequent thereto that enables the target cell amount to be obtained. Specifically, the plan creation unit 144 references the cell DB 50 and the protocol DB 52, and identifies the type of expansion passage process and the type of culture process subsequent thereto. The information to identify the type of expansion passage process is the protocol ID. The protocol data of the expansion passage process is data including information about the type and number of containers for seeding. The type of container identified is identified together with the size of container. Namely, not only the shape pattern and substance of the container, but the size of container is also a factor that makes containers a different type. The plan creation unit 144 make a plan to perform the expansion passage process and the culture process plural times when this is needed to obtain the target cell amount. Moreover, the plan creation unit 144 may create a combination of expansion passage process type and culture process type by selecting from out of a collection of pre-prepared combination patterns, or by calculating each time.

Next, at step S16, the plan creation unit 144 may plan so as to perform a thawing process and an initial culture process in the time period between the culture start time and the first most expansion passage process, and to perform a maintenance passage process and subsequent culture process an appropriate number of times. The thawing process and initial culture process are not required when target cells are those subjected to a repeated succession of passage processes.

Next, at step S18, the plan creation unit 144 displays the created cell culture plan on the display 54, and prompts confirmation or amendment by a user. The plan creation unit 144 amends the cell culture plan according to amendment instruction when an amendment instruction has been received from a user. The plan creation unit 144 confirms the cell culture plan and ends the cell culture plan creation processing when confirmation has been received from a user.

Description follows regarding a first case to a third case as cases related to creating a cell culture plan by the cell culture plan creation device 40 according to the first exemplary embodiment. First description follows regarding common items to each of the cases.

In the passage processes for each of the cases, up to 1/2 of the cell amount after the immediately preceding culturing is employed for seeding. Namely, the maximum passage utilization rate is 0.5. Adopting this approach enables subsequent processing to be performed even in cases in which the post-culture cell amount has not increased as much as expected. This means that a maintenance passage process to seed a cell amount of one unit may be executed when the post-culture cell amount of the immediately preceding culture process has exceeded two units, or in cases in which there is no rush to transition to an expansion passage process, may be executed after waiting until the post-culture cell amount has exceeded four units, for example. In such cases the maintenance passage process occurrence number can be reduced, enabling experiment facilities that would have been required therefor, such as the workbench, to be utilized for another experiment. In the first case and third case, described later, a plan is made so as to perform a maintenance passage process after waiting until the post-culture cell amount has exceeded four units. The maximum passage utilization rate is not limited to being 0.5, and a configuration may be adopted in which an appropriate value can be instructed by a user.

Moreover, in each of the following cases, the cell utilization rate for another experiment after the final time of culturing is 0.5, the same as the maximum passage process utilization rate. This means that in cases in which the usable cell amount after the final time of culturing is eight units, the post-culture cell amount needs to be 16 units. The cell utilization rate after the final time of culturing may be configured so as to be a different value instructed by a user.

Moreover, in each of the following cases, the seeding cell amount per single container in a maintenance passage process is 1 unit, however this may be configured to enable different values to be instructed by a user.

First Case

In the first case the whole time period is 15 days, and the target cell amount is eight units.

The post-culture cell amount is found from the following equation.

$$\text{Post-culture cell amount} = \text{pre-culture cell amount} \times \mu^{t}$$

wherein $\mu$ is the multiplication rate, and t is the culture time period. For example, when $\mu$ = 1.6 and the pre-culture cell amount = 1 unit then the post-culture cell amounts for each of the culture time periods are as set out below.

Table 1

| Culture Time Period (Days) | 1 | 2 | 3 | 4 |
|---|---|---|---|---|
| Post-Culture Cell Amount (Units) | 1.6 | 2.56 | 4.1 | 6.6 |

Note that in the above case a multiplier for one day is employed as the multiplication rate $\mu$, however a multiplier for one hour may be employed, so as to determine culture time periods in one-hour units instead of one-day units. In such cases, although this results in a passage process sometimes being performed in a time band when people are not working, such as during the night, or in a time band when short staffed or the like, this is not an issue when the passage process is performed by full automation using a robot system (see the second and third exemplary embodiments).

An expansion passage process is performed by extracting cells from one container and seeding one unit in each of two containers, combined with a culture process in which one unit's worth of seeded cells is proliferated to 4.1 units in three days, with this combination repeated for two stages. The post-culture cell amount obtained as a result thereof is 16.4 units, with a plan to obtain eight units of usable cells therefrom. Six days are taken up by the expansion passage process and culture process.

The plan for the remaining nine days is to perform an initial culture process of three days after first performing a thawing process, then performing a combination of a maintenance passage process followed by three days of a culture process twice. If the remaining time period is something other than nine days, then the number of times that the combination of a maintenance passage process followed by a culture process is performed, and the culture time period, may be adjusted as appropriate so to as to match the remaining time period.

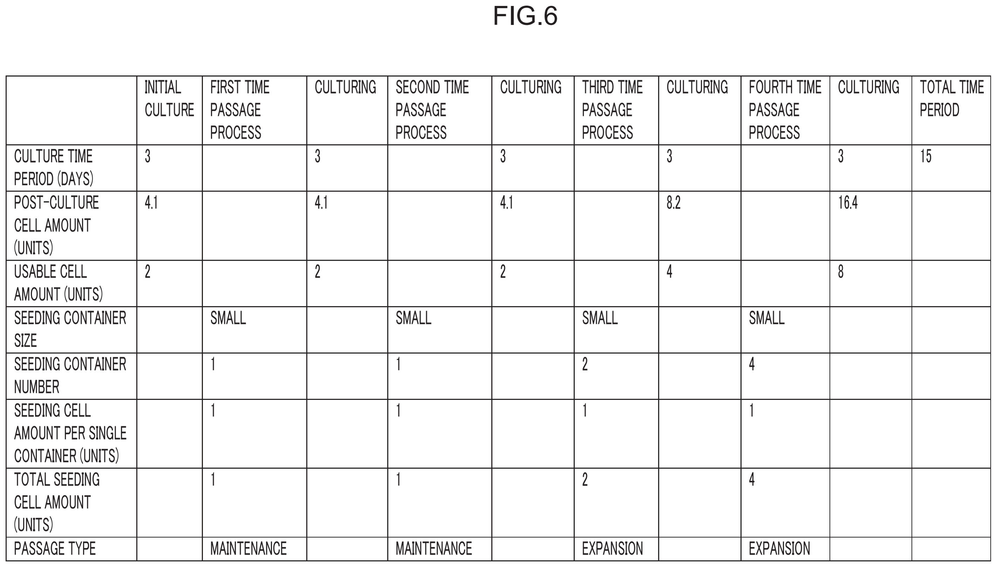

Fig. 6 schematically illustrates an example of a cell culture plan of the first case. In the example of Fig. 6, the passage process plan identifies seeding container size, seeding container number, seeding cell amount per single container, total seeding cell amount, and passage type. Moreover, a plan for a culture process following on from a passage process identifies a culture time period, a post-culture cell amount, and a usable cell amount. Moreover, the total time period that is the total of the culture time periods (the same as the whole time period) is also identified. In addition to the information of Fig. 6, a cell ID of processing target cells, a protocol ID for each process identified by the plan creation unit 144, and the culture end time, are contained in the cell culture plan. The start time of each passage process is identified based on culture start time or culture end time, and on the culture time period of each culture process.

Although container size is illustrated in the created plan of the example of Fig. 6, the type of container instructed in the protocol data may be indicated instead of, or as well as, this. For clarity purposes the container size in indicated on the display. Note that for the container size, "small" (a size so as not to become confluent even when proliferated to about two units) may be selected when culturing so as to extract one unit after culturing in one container, "medium" (a size so as not to become confluent even when proliferated to about four units) may be selected when culturing so as to extract two units after culturing in one container, and "large" (a size so as not to become confluent even when proliferated to about six units) may be selected when culturing so as to extract three units after culturing in one container.

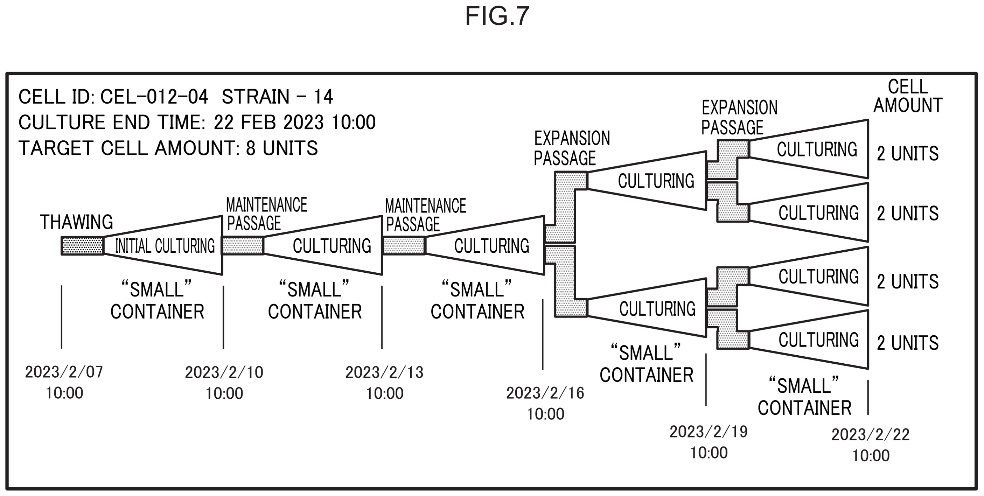

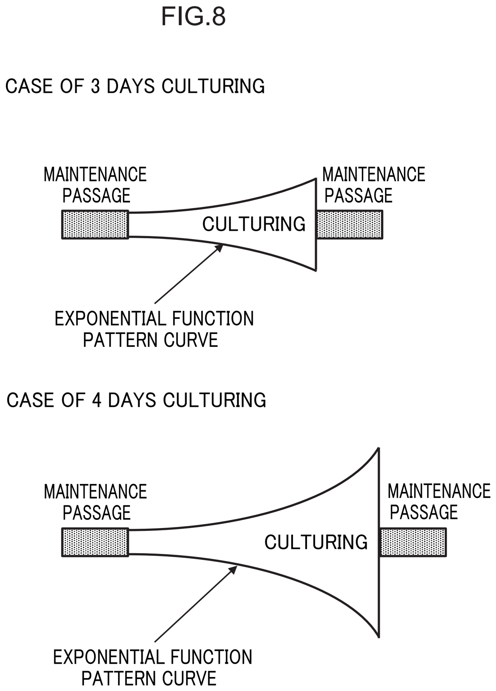

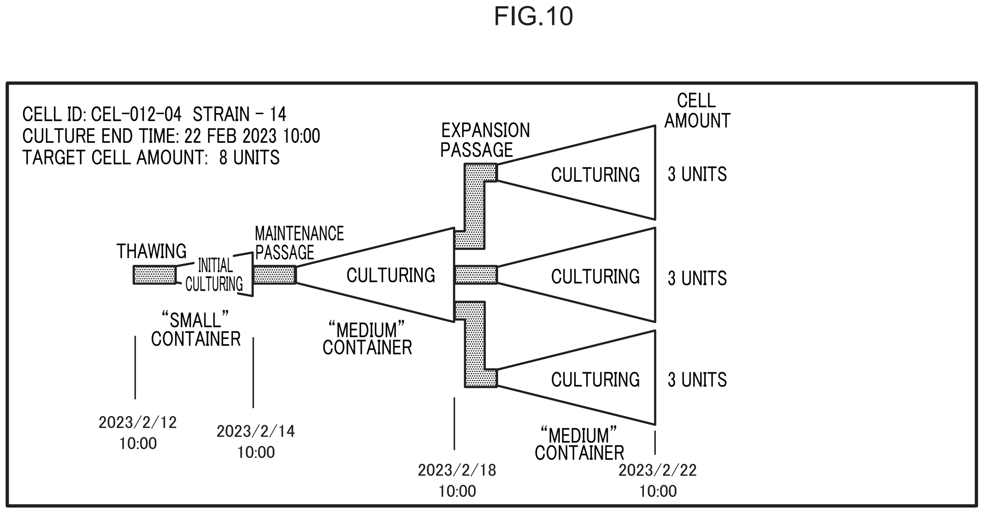

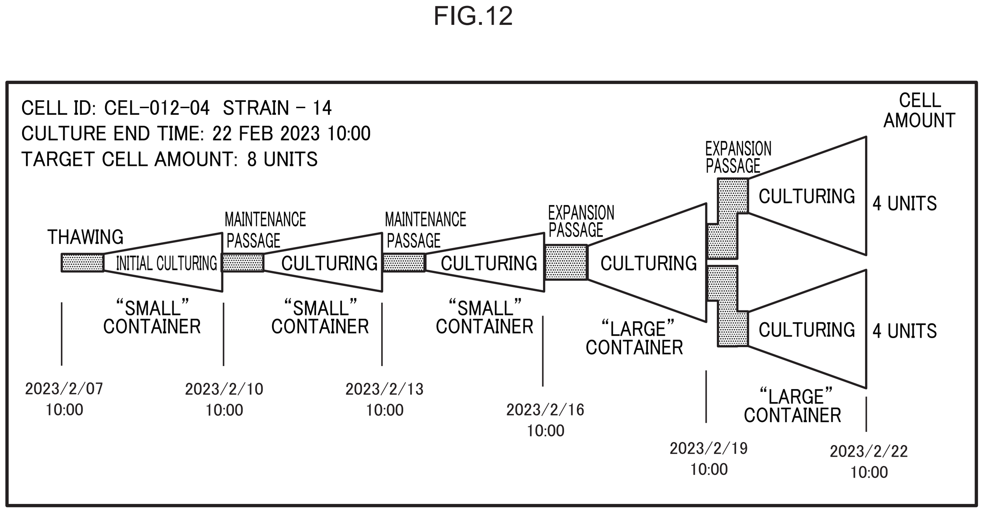

A display example of the cell culture plan of the first case is illustrated in Fig. 7. The graphics illustrated by shading are first graphics representing passage processes, the horizontally oriented trapezoidal graphics are second graphics representing culture processes. The horizontal direction is an example of the first direction, and the vertical direction is an example of the second direction. The first direction corresponds to elapse of time, with the graphics corresponding to each process arranged in order of processing (from left to right in a time series), with the respective horizontal widths of graphic for each process corresponding to the time period needed for each process. Moreover, the second direction represents increase/decrease in the cell amount. Similar applies to Fig. 10 and Fig. 12 below. Note that in the example of Fig. 7 the second graphics illustrating the culture processes are graphics in which the cell amount is illustrated as increasing linearly, however as illustrated in Fig. 8, the cell amount may be represented so as to increase exponentially using a curved line with the shape of an exponential function.

Second Case

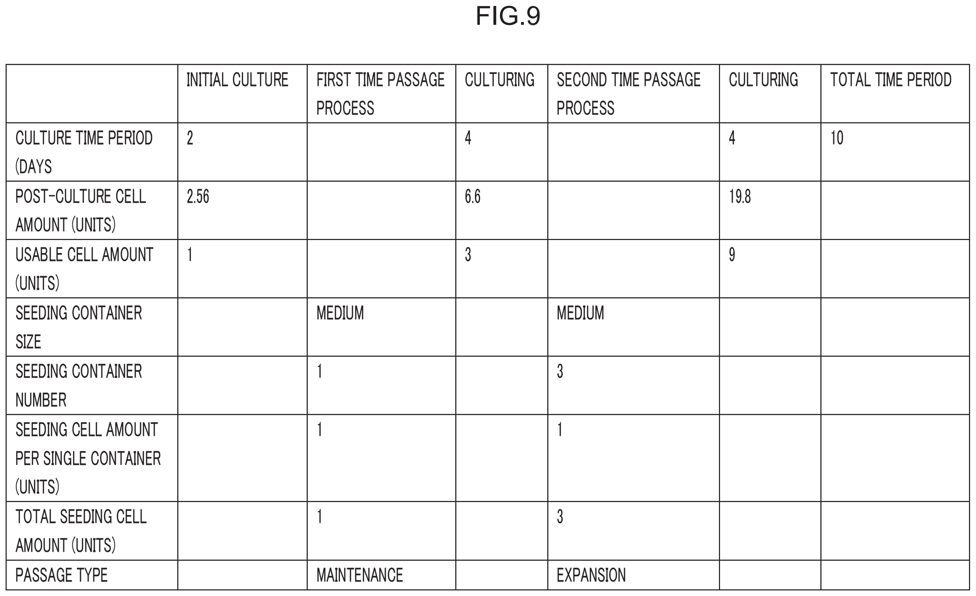

The state of cells is preferably stabilized by performing a maintenance passage process and a culture process a number of times after thawing cells due to there sometimes being cases in which the state of the cells in unstable, such as there being a high mortality rate of the cells or the like. However, there are cases when there is a desire for priority to be given to proliferating to the required cell amount in a short time period. The second case is an example where priority is placed on shortening the whole time period. The target cell amount is eight units, the same as in the first case, however the whole time period is 10 days, i.e. shorter than in the first case. Fig. 9 illustrates an example of a schematic cell culture plan of the second case, and Fig. 10 illustrates a display example of the cell culture plan of the second case.

A combination of extracting cells from one container and seeding one unit in each of three containers in an expansion passage process, combined with a culture process in which the one unit's worth of seeded cells is proliferated to 6.6 units in four days, is performed once. This results in a plan to obtain a post-culture cells of 19.8 units, so as to obtain 9 units of usable cells therefrom. Note that because the target cell amount is eight units, obtaining a cell amount of at least this, i.e. nine units, is not an issue. Four days are taken for this expansion passage process and culture process.

The plan for the remaining six days is to perform an initial culture process after first performing a thawing process, then to perform a combination of a maintenance passage process and culture process once. Due to the remaining time period being shorter than six days, the initial culture process is shortened from three days to two days compared to the first case, and the maintenance passage process and the subsequent culture process are reduced from being performed twice to being performed once. However, the time period of the culture process after the maintenance passage process is made four days rather than three days in order to obtain three units as the usable cell amount at the point in time when the expansion passage process is performed.

Suppose that a cell culture plan capable of obtaining a target cell amount of eight units in the shortest time period is the second case, then as long as the whole time period is less than ten days, the cell culture plan creation device 40 may propose a plan in which more frozen cells are employed in a thawing process, and a greater cell amount is cultured at the same time.

Third Case

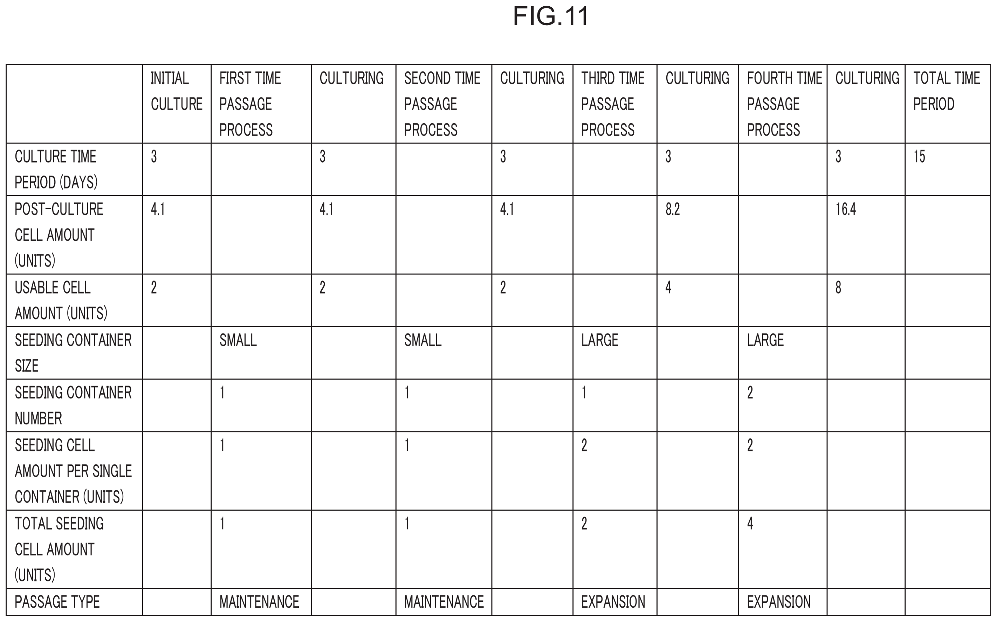

The third case is, similarly to the first case, a plan in which the whole time period is 15 days, the target cell amount is eight units, and the expansion passage process of the first case is substituted by a process to seed two units in a single container. Fig. 11 illustrates an example of a schematic cell culture plan of the third case, and Fig. 12 illustrates a display example of a cell culture plan of the second case.

Although the container sizes were all "small" in the first case, a "large" size container needs to be employed after the expansion passage processes in the third case. Adopting such an approach means that even though the cell culture plan is to obtain the same cell amount, plural different plans may be created therefor, and a user may select which plan to actually employ in consideration of various factors. Moreover, for example, if employing "small" size containers as much as possible has been received in advance as the wishes of a user, then a plan can be presented so as to match the user's wishes.

The cell culture plan creation device according to the first exemplary embodiment is accordingly able to create a plan to culture cells of a required amount in cases in which, for example, cells of a specific amount are required for a follow-on experiment.

Second Exemplary Embodiment

Description follows regarding a case in a second exemplary embodiment in which processing of cell culturing is executed by a robot system according to a cell culture plan created by a cell culture plan creation device. Note that in the second exemplary embodiment the same reference numerals will be appended across the drawings to the same or equivalent configuration elements and parts to those of the first exemplary embodiment, and detailed explanation thereof will be omitted.

A cell culture system according to the second exemplary embodiment envisages a system to automate experiments handling cells in an environment similar to an experiment environment utilized by a researcher. Note that reference to "an experiment" in the present exemplary embodiment is not limited to something performed with an objective of finding out a result of manipulation or processing on a target such as cells or the like, and encompasses cases in which manipulation or processing is performed on a target, without investigating a result thereof. For example, a cell thawing process, passage process, and culture process are also called an experiment.

A typical example of an experiment environment utilized by a researcher is an environment with a workbench installed to perform most experiment work, as well as locations somewhere away from the workbench installed with a cell observation device, a cell measurement device, a centrifuge, and the like. When a researcher performs experiment work, the researcher performs experiment work at the workbench, and also, as required during performing the experiment work, moves between the workbench and the cell observation device, the cell measurement device, the centrifuge, or the like by walking carrying a specimen container in order to use these devices.

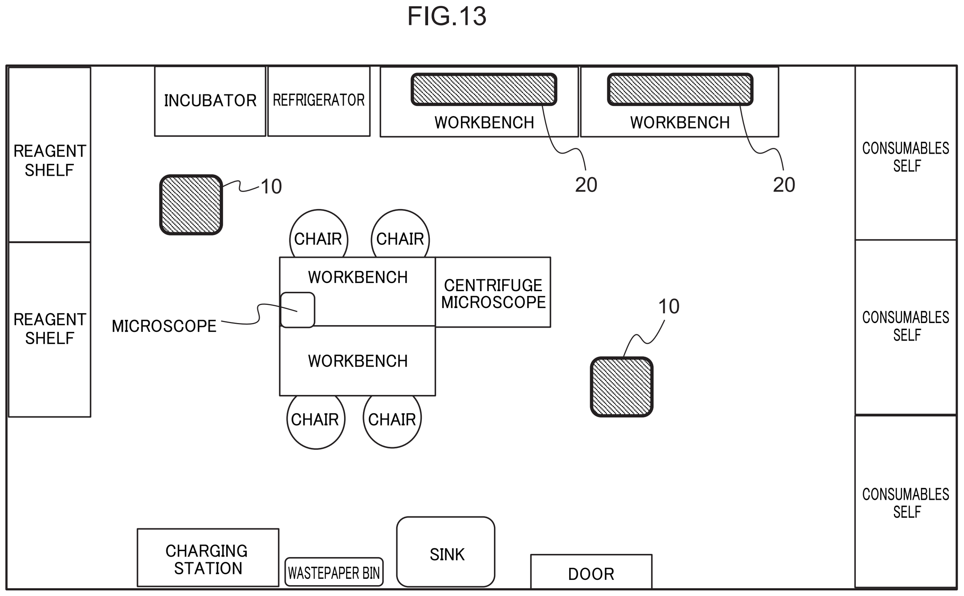

The cell culture system according to the present exemplary embodiment runs in an environment similar to the typical example of an experiment environment utilized by a researcher as described above. Fig. 13 is a schematic plan view illustrating an example of an experiment environment for running a cell culture system according to the present exemplary embodiment. In the example of Fig. 13, in addition to the above cell observation device, cell measurement device, centrifuge, and workbench, the experiment environment also includes experiment equipment such as reagent shelves, an incubator, a refrigerator, consumables shelves, a charging station, a sink, chairs, and the like. Note that a microscope in Fig. 13 is an example of the cell observation device and the cell measurement device referred to above.

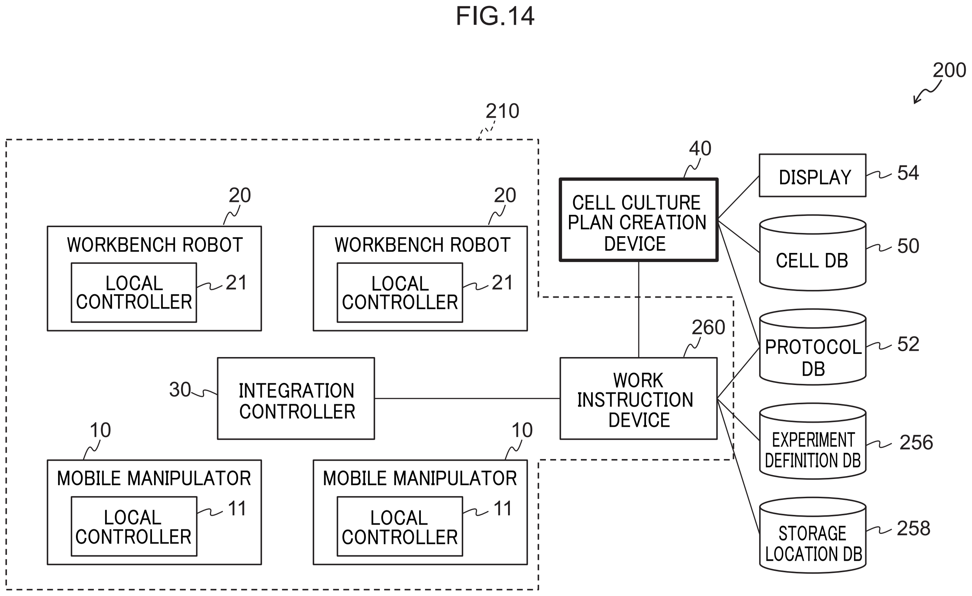

As illustrated in Fig. 14, a cell culture system 200 according to the second exemplary embodiment includes a cell culture plan creation device 40 and a robot system 210.

The cell culture plan creation device 40 is similar to the cell culture plan creation device 40 of the first exemplary embodiment. In the second exemplary embodiment too, the cell culture plan creation device 40 is connected to a cell DB 50, a protocol DB 52, and a display 54.

The robot system 210 includes mobile manipulators 10, workbench robots 20, an integration controller 30, and a work instruction device 260. The work instruction device 260 is connected to each of an experiment definition DB 256, a protocol DB 52, and a storage location DB 258. The protocol DB 52 is shared with the cell culture plan creation device 40. Note that although there are two mobile manipulators 10 and two workbench robots 20 illustrated in the example of Fig. 14, there is no limitation to there being two thereof. There may be a single, or three or more, of the mobile manipulator 10 and the workbench robot 20.

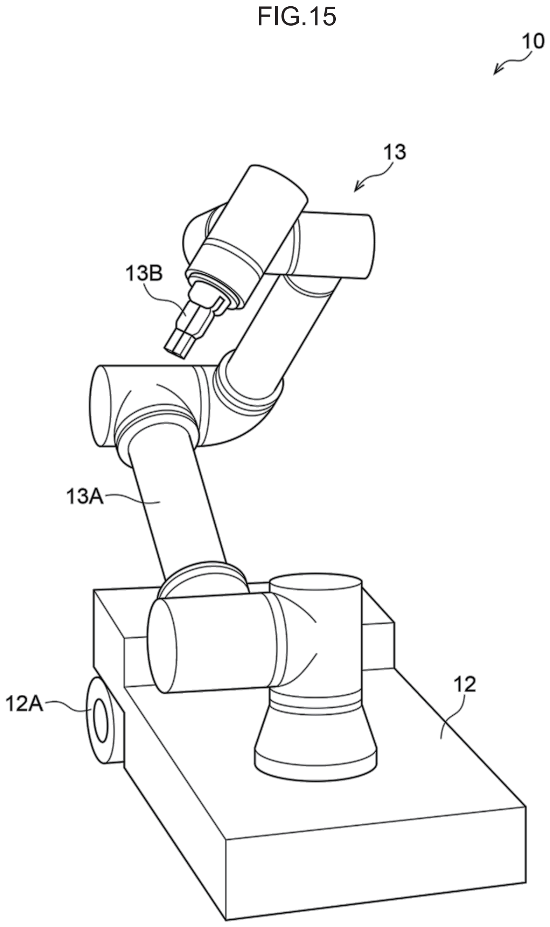

The mobile manipulators 10 each include a local controller 21, a moving mechanism to move across the floor, and a gripping mechanism to grip an object. Fig. 15 is a perspective view of the external appearance of the mobile manipulator 10. As illustrated in Fig. 15, the mobile manipulator 10 includes a trolley 12 and a robot arm 13.

The trolley 12 is an example of a moving mechanism, and includes two drive wheels 12A driven under control of a local controller 11. The trolley 12 also includes variable direction casters on a bottom face at the nearside in Fig. 15. The trolley 12 is able to move freely over the floor similarly to a researcher by the two drive wheels 12A being driven independently from each other. The moving mechanism is not limited to being two independently driven wheels, and may be any freely selected mechanism capable of moving over the floor, and is preferably a mechanism having few limitations in the way of moving including turning. The robot arm 13 is an example of a gripping mechanism and is mounted on the trolley 12. The robot arm 13 includes an arm 13A, and a hand 13B attached to a distal end of the arm 13A. The arm 13A may be an articulated arm provided with a configuration to change the position and pose of the hand 13B in three dimensional space with, for example, six degrees of freedom. The hand 13B is, for example, a two fingered robot hand capable of gripping a container or the like. Note that although the mobile manipulator 10 illustrated in Fig. 15 is an example equipped with a single robot arm 13, a two handed configuration equipped with two robot arms 13 may be adopted.

The mobile manipulator 10 includes a non-illustrated vision sensor at a site near to the hand 13B of the arm 13A. The vision sensor is a sensor for recognizing objects in the periphery of the mobile manipulator 10, and gripping target objects for gripping with the hand 13B, such as a container or the like. The vision sensor is configured including a camera and an image processing device, and recognizes peripheral objects and gripping target objects by using the image processing device to perform image processing on images captured with the camera. The mobile manipulator 10 is accordingly able to move autonomously. Note that the sensor for recognizing peripheral objects and gripping target objects is not limited to being a vision sensor, and a laser radar or the like capable of measuring a three-dimensional position of each point in the periphery may be employed. A sensor for recognizing peripheral objects may also be mounted to the trolley 12.

The local controller 11 controls operation of each motor of the mobile manipulator 10 so as to be able to implement instructions of the integration controller 30. Specifically, the local controller 11 controls the operation of the mobile manipulator 10 based on recognition results output from the vision sensor, instructions from the integration controller 30, and the like. More specifically, the local controller 11 controls the operation of the mobile manipulator 10 so as to grip a gripping target object such as a container or the like with the hand 13B, and to transport the gripping target object that has been gripped to a specific position, such as the workbench or the like.

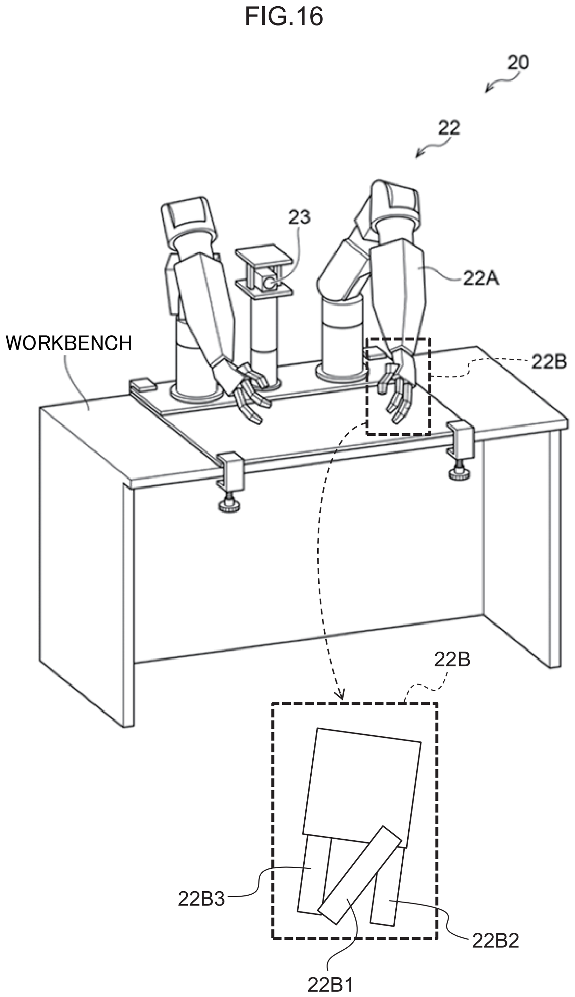

The workbench robots 20 are each fixed to a workbench, and equipped with the local controller 21, and a gripping mechanism for gripping objects on the workbench. Gripping target objects gripped by the workbench robot 20 include, as well as containers, an aspirator, an electronic pipette, an electronic micro pipette, or the like for performing suctioning cells and the like inside a container or discharging cells or the like to inside the container. Fig. 16 is a perspective view of the external appearance of the workbench robot 20. As illustrated in Fig. 16, the workbench robot 20 is equipped with two robot arms 22, and a vision sensor 23.

The robot arms 22 are each an example of a gripping mechanism, and include an arm 22A, and a hand 22B attached to a distal end of the arm 22A. The arm 22A may be an articulated arm including a configuration to change the position and pose of the hand 22B in three-dimensional space with, for example, six degrees of freedom. The hand 22B is, for example, a three-fingered robot hand capable of gripping a container or the like and including joints on each finger.

An expanded schematic diagram of the hand 22B is illustrated at the bottom of Fig. 16. In this schematic diagram the joints of each of the fingers of the hand 22B are omitted from illustration, and a simplified view of the shape and arrangement of each of the fingers is illustrated. The hand 22B includes a first group of fingers including a first finger 22B1, and a second group of fingers including a second finger 22B2 and a third finger 22B3, and is a structure capable of gripping an object between the opposing first group and second group. Corresponding to human fingers, the first finger 22B1 works as a thumb, the second finger 22B2 works as an index finger, and the third finger 22B3 works as a middle finger. Note that the hand 22B may be a robot hand with four or more fingers. Namely, the first group may include a finger other than the first finger 22B1, and/or the second group may include a finger other than the second finger 22B2 and the third finger 22B3. Each of the fingers of the hand 22B preferably includes two joints partway along. This thereby enables wrapping around so as to grip the external shape of an object stably, and facilitates fine manipulation such as pressing a button or an experiment instrument or the like.

In the following the two hands 22B of the robot arms 22 will be denoted as a left hand 22BL and a right hand 22BR when discriminating in the description between left and right hands. Similarly for the fingers of the hand 22B, the fingers of the left hand 22BL will be denoted as first finger 22B1L, second finger 22B2L, and third finger 22B3L, and the fingers of the right hand 22BR will be denoted as first finger 22B1R, second finger 22B2R, and third finger 22B3R.

The vision sensor 23 is mounted to a pan tilt mount, and is a sensor for recognizing gripping target objects such as a container or the like arranged on the workbench.

The local controller 21 controls the operation of each motor of the workbench robot 20 so as to enable implementation of an instruction from the integration controller 30. Specifically, the local controller 21 controls operation of the workbench robot 20 based on recognition results output from the vision sensor 23, and instructions and the like from the integration controller 30. More specifically, the local controller 21 controls operation of the workbench robot 20 so as to execute experiment processes in which cells are handled by coordinating the two robot arms 22. For example, the local controller 21 controls operation of the workbench robot 20 so as to grip a container with the hand 22B of one of the robot arms 22, to grip a pipette with the hand 22B of the other robot arm 22, and to execute processes such as suctioning liquid inside the container.

Note that although in Fig. 16 an example is illustrated in which the workbench robot 20 is fixed directly to the workbench, the workbench robot 20 may have a fixed relative positional relationship to the workbench by, for example, being fixed to the floor, a wall, the ceiling, or the like.

Based on a work instruction from the work instruction device 260, the integration controller 30 interlinks the local controllers 11 of the mobile manipulators 10 and the local controllers 21 of the workbench robots 20 so as to control each operation of the mobile manipulator 10 and the workbench robots 20. Specifically, the integration controller 30 executes an experiment with the mobile manipulators 10 and the workbench robots 20 including transporting and placing a container containing cells between the workbench and other experiment equipment.

Gripping operations and manipulations of experiment equipment by each of the mobile manipulators 10 are implemented by the local controller 11 executing a pre-taught operation plan with the mobile manipulator 10 based on recognition results output from the vision sensor. Similar applies to gripping operations and manipulations of experiment equipment by the workbench robots 20.

Moreover, movement of each of the mobile manipulators 10 is implemented by the local controller 11 moving the mobile manipulator 10 to a position of experiment equipment as instructed by the integration controller 30 based on a pre-saved layout map of the experiment environment. Note that the local controller 11 moves the mobile manipulator 10 such that movement to a target position is performed while avoiding obstacles based on the recognition results output from the vision sensor.

Note that the hardware configurations of the integration controller 30, the local controller 11 of the mobile manipulator 10, the local controller 21 of the workbench robot 20, and the work instruction device 260 are each substantially the same as the hardware configuration of the cell culture plan creation device 40 illustrated in Fig. 1 and so explanation thereof will be omitted.

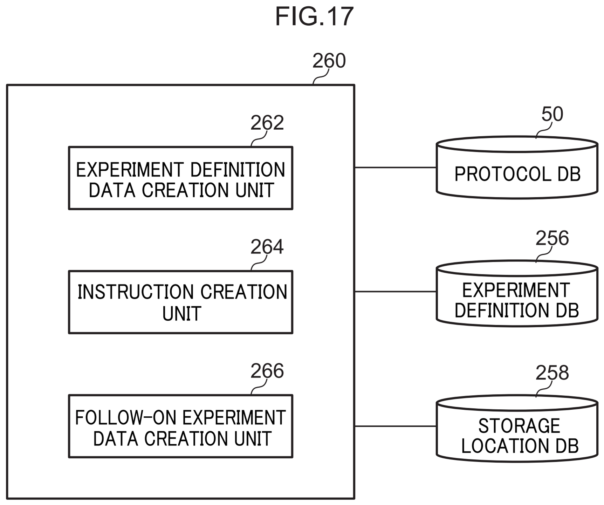

Next, description follows regarding a functional configuration of the work instruction device 260. Fig. 17 is a block diagram illustrating an example of a functional configuration of the work instruction device 260. As illustrated in Fig. 17, the work instruction device 260 includes, as functional configuration, an experiment definition data creation unit 262, an instruction creation unit 264, and a follow-on experiment data creation unit 266. Each of the functional configuration is implemented by a CPU reading a work instruction program stored on a storage device and expanding and executing the work instruction program in memory.

The experiment definition data creation unit 262 acquires preparation transport information and a cell culture plan from the cell culture plan creation device 40. The preparation transport information is information needed to take cells that are the experiment target out from a storage location. For example, when the cell culture plan starts with a thawing process, the preparation transport information includes a container ID that is identification information of a container that is the target of the thawing process, a container storage location ID that is identification information of the storage location for the container, or the like. Moreover, for example, in cases in which the cells are already being cultured inside an incubator, the preparation transport information contains a container ID of the container that is to be the target for the first most passage process, the container storage location ID, and the like. The preparation transport information may be configured by the input unit 142 of the cell culture plan creation device 40 receiving information input by a user.

As described in the first exemplary embodiment, a first execution instruction for a passage process, and a second execution instruction for a culture process, are included in the cell culture plan. Moreover, as required, a third execution instruction for a thawing process may also be included therein. The first execution instruction contains information to identify the type of passage process (protocol ID), and information to identify the timing to execute the passage process (start date and time etc.). Moreover, the cell culture plan also includes a cell ID of the processing target cells, and a cell ID classification number.

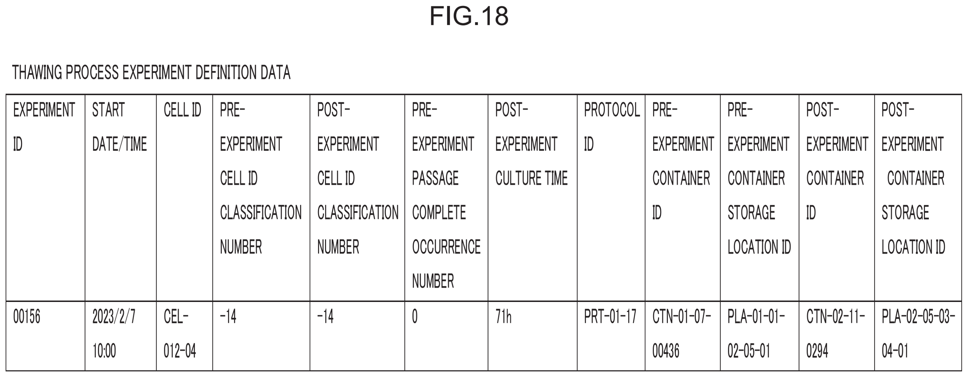

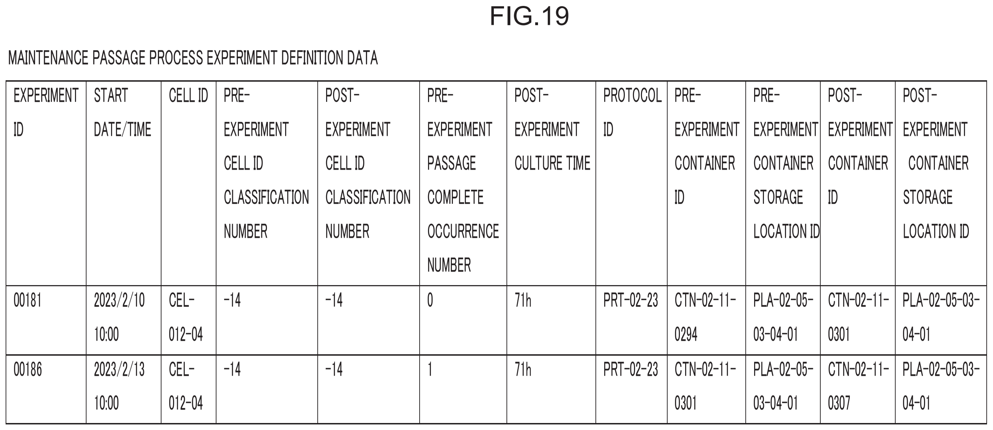

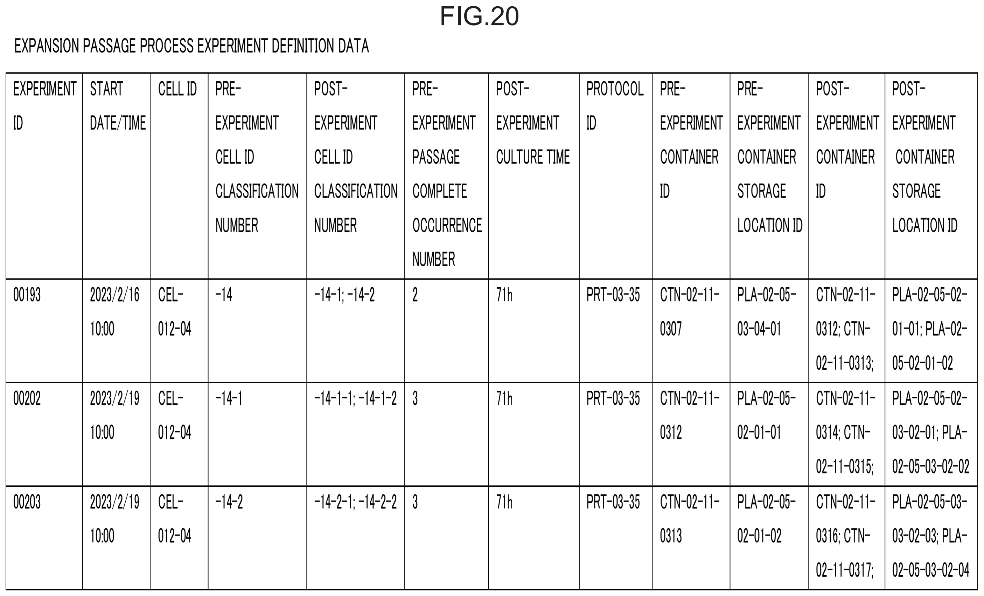

The experiment definition data creation unit 262 creates experiment definition data, for example such as that illustrated in Fig. 18 to Fig. 20, based on the acquired information. Fig. 18 illustrates experiment definition data for a thawing process, Fig. 19 illustrates experiment definition data for a maintenance passage process, and Fig. 20 illustrates experiment definition data for an expansion passage process.

Specifically, the experiment definition data creation unit 262 appends an experiment ID that is an identification number for the experiment to each process (experiment), and also creates information for each item in the experiment definition data based on the information included in the preparation transport information and the cell culture plan. Each ID may be appended automatically based on a predetermined rule, or input or amendment of each ID may be received manually.

Moreover, the notation format of the cell ID classification number is "-n-o-p-...". n, o, p, ... are appended in sequence each time cells are divided into plural containers in an expansion passage process, so as to update the cell ID classification number with which number of container the cells are in when cells have been divided into many containers. The example of Fig. 18 indicates n = 14, with this indicating that the cells that have been thaw processed are cells of the 14th container from out of cells prior to freezing that were divided into many containers.

The notation format of the pre-experiment passage complete occurrence number is "m". m is incremented when performing a passage process irrespective of whether or not this is a maintenance passage or an expansion passage. Fig. 18 illustrates the passage complete occurrence number after a thawing process, however in which a passage process has been performed prior to thawing then it is this passage process occurrence number that is included.

Moreover, the notation format of the container ID is "CTN-cc-dd-eeeee". CTN is a text string indicating that it is a container ID, cc is a number representing a course classification of container type and, for example, 01 is a conical tube, 02 is a square flask, and the like. dd is a number representing a fine classification of container type and, for example, is a number or the like associated with a container product number. eeee is an individual number of a container. The container ID may be acquired by reading an optical readable code such as a barcode, or a radio frequency identification (RFID) tag or the like attached to the container. A position of a container identified by the container ID may be tracked, such that the container ID is acquired from the current position of the container.

The notation format of the container storage location ID is "PLA-ff-gg-hh-ii-jj". PLA is a text string indicating that it is a container storage location ID, ff is a number representing a type of storage location and, for example, 01 is a refrigerator, 02 is an incubator, 03 is a storage shelf, 04 is a table, and the like. gg is a number to discriminate between storage locations of the same type and, for example, 03 is machine No. 3 (refrigerator No. 3 when ff is 01 refrigerator). hh is a number expressing an area in the storage location where the container rack is placed, a number expressing an area in the storage location where the container is placed when a container rack is not used, and, for example, is a shelf number. ii is a number expressing a position inside the area where a container rack is placed, or is a number expressing a position inside an area where the container is placed when a container rack is not used, and, for example, is a number representing a position on a shelf. jj is a number expressing a position of a container in a container rack, and, for example, is 00 when a container rack is not used.

Storage location data associated with the container storage location ID and a position of the storage location are stored in the storage location DB 258. The storage location position is represented by a combination of a position of equipment such as a refrigerator, an incubator, a storage shelf, or a table that is the storage location, and a position inside the equipment. The position of the equipment is, for example, expressed by coordinates set in a room where the equipment is placed. The position inside the equipment is, for example, represented by coordinates set within each equipment. The physical location of the storage location DB 258 may be within the same storage device as the one where the experiment definition DB 256 is stored, or may be within a separate storage device. For example, the storage location DB 258 may be stored in a storage device inside the integration controller 30. In cases in which the storage location DB 258 is not stored inside the integration controller 30, the integration controller 30 may acquire the storage location data from a storage location DB via the work instruction device 260, or may, for example, acquire the storage location data directly from the storage location of the storage location DB 258 using wireless communication, without going through the work instruction device 260.

The post-experiment container ID and the post-experiment container storage location ID may be included in the cell culture plan, or may be configured so as to be received by input from a different user, or may be configured so as to be identified by a predetermined rule. Moreover, in Fig. 18 to Fig. 20, the post-experiment culture time is 71 hours, with the culturing time being 71 hours because it is estimated that a time required for a passage process is one hour from out of three days (72 hours) of culture time period.

The instruction creation unit 264 creates a work instruction such as a preparation transport instruction for the controller to control operation of the mobile manipulators 10, or a workbench experiment instruction for the controller to control operation of the workbench robots 20. The "controller" referred to here is a controller that can be given a work instruction from the work instruction device 260, and so corresponds to the integration controller 30 in the exemplary embodiment of Fig. 14. The operation of the mobile manipulator 10 is controlled stepwise by the integration controller 30 and the local controller 11, and so the integration controller 30 also corresponds to a controller to control operation of the mobile manipulator 10. Moreover, the operation of the workbench robot 20 is controlled stepwise by the integration controller 30 and the local controller 21, and so the integration controller 30 also corresponds to a controller to control operation of the workbench robot 20.

The preparation transport instruction is a work instruction created based on the preparation transport information for taking cells that are the experiment target from the first storage location and transporting them to the workbench. For example, the preparation transport instruction includes an instruction related to movement of the mobile manipulator 10, and an instruction related to manipulation of the mobile manipulator 10. The instruction related to movement of the mobile manipulator 10 includes, for example, information about a position of the movement destination. The instruction related to manipulation of the mobile manipulator 10 includes, for example, information for gripping a target object, and information for placing the gripped target object. The information for gripping the target object includes, for example, information to identify a container and information to identify a location of the container when the gripping target object is a container. The information for placing the target object includes, for example, information of a position on the workbench for placing the target object.

The workbench experiment instruction is created based on the workbench experiment identifying information and is a work instruction to execute an experiment on transported cells.

The instruction creation unit 264 may, as the work instruction, create a storage transport instruction for a controller to control operation of the mobile manipulator 10. The storage transport instruction is created based on the storage transport information, and is an instruction to transport the cells to the second storage location after the experiment has been performed on the workbench. The reference here to "controller" means a controller that that can be given the storage transport instruction from the work instruction device 260, and so this applies to the integration controller 30 in the exemplary embodiment of Fig. 14. The operation of the mobile manipulator 10 is controlled stepwise by the integration controller 30 and the local controller 11, and so the integration controller 30 also corresponds to the controller for controlling operation of the mobile manipulator 10.

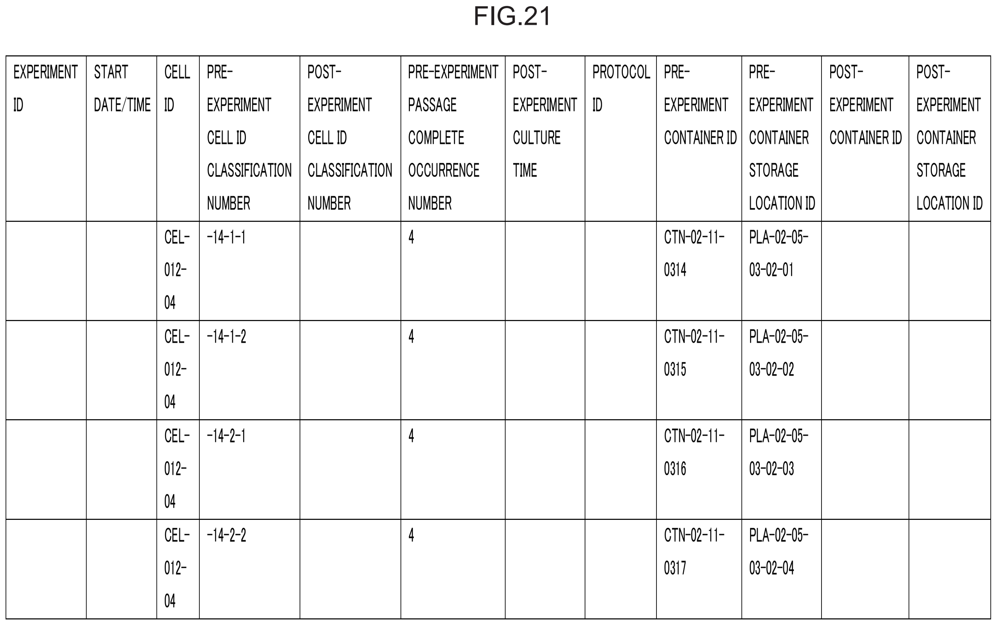

A follow-on experiment data creation unit 266 creates data for a follow-on experiment containing data identifying a container holding cells to be utilized in the follow-on experiment or data identifying a second storage location for storing a container holding the cells (hereafter referred to as "follow-on experiment data"). Fig. 21 illustrates an example of follow-on experiment data.

The follow-on experiment data may include both the data identifying the container holding the cells and also data identifying the second storage location for storing the container holding the cells, or may include one of these types of data. Management is performed so as to be able to identify the second storage location from data identifying the container in cases in which data of the second storage location is not contained. For example, which container has been received in which storage location may be recoded in a separately prepared system for managing storage locations at the point in time when a container has been received at a second storage location. This thereby enables a storage location of such a container to be found from information to identify the container when a follow-on experiment is to be performed.

Moreover, a container stored with post-experiment cells may be supplied to the follow-on experiment by being placed in a second storage location within the reach of a hand of the workbench robot 20 without being transported by the mobile manipulator 10. In such cases, there is no need to perform preparation transport using the mobile manipulator 10 in the follow-on experiment as long as the follow-on experiment is to be performed at the same workbench.

Next, description follows regarding operation of a cell culture system 200 according to a second exemplary embodiment.

The cell culture plan creation device 40 executes the cell culture plan creation processing as illustrated in Fig. 5, and also receives preparation transport information. The cell culture plan creation device 40 transmits the preparation transport information and the cell culture plan to the work instruction device 260.

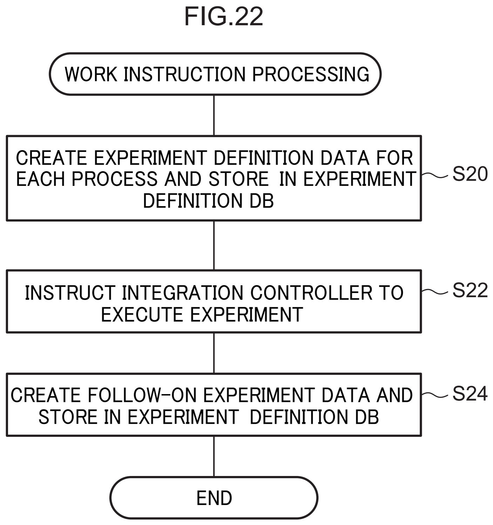

The work instruction device 260 executes the work instruction processing illustrated in Fig. 22.

At step S20, the experiment definition data creation unit 262 acquires the preparation transport information and the cell culture plan transmitted from the cell culture plan creation device 40, creates experiment definition data for each process based on the acquired preparation transport information and cell culture plan, and stores this in the experiment definition DB 256.

Next, at step S22 the instruction creation unit 264 acquires from the protocol DB 52 the protocol data as identified by the protocol ID contained in the experiment definition data stored in the experiment definition DB 256, and creates a work instruction for each process based on the protocol data. The instruction creation unit 264 transmits the created work instruction to the integration controller 30 so as to match the start date and time included in the experiment definition data, thereby instructing the integration controller 30 to perform experiment execution.

Next, at step S24, a follow-on experiment data creation unit 266 creates follow-on experiment data based on the experiment definition data. Specifically, the follow-on experiment data creation unit 266 carries forward the cell ID contained in the experiment definition data to the follow-on experiment data. Moreover, the follow-on experiment data creation unit 266 takes a post-experiment cell ID classification number, a post-experiment container ID, and a post-experiment container storage location ID contained in the experiment definition data, and creates follow-on experiment data of a pre-experiment cell ID classification number, a pre-experiment container ID, and a pre-experiment container storage location ID. The follow-on experiment data creation unit 266 then stores the created follow-on experiment data in the experiment definition DB 256, and ends the work instruction processing.

The integration controller 30 that has received the work instruction creates an operation command to control the mobile manipulator 10 and the workbench robot 20 based on the work instruction, and controls the mobile manipulator 10 and the workbench robot 20 via the local controller 11 and the local controller 21.

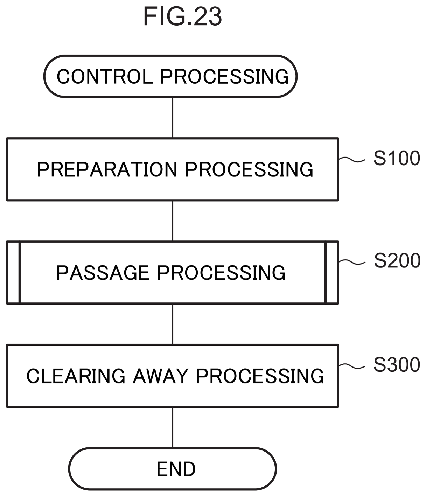

Description follows regarding control processing by the integration controller 30 for an example of a passage process. Fig. 23 is a flowchart illustrating a flow of control processing executed by a CPU of the integration controller 30. Description follows regarding an example of adhesion culture.

At step S100, as preparation processing for the passage process, the integration controller 30 uses the mobile manipulator 10 to take a first container containing cells being cultured out from a first incubator and to transport the first container onto a workbench. The first container is, for example, a square flask or the like.

Specifically, the integration controller 30 instructs the local controller 11 of the mobile manipulator 10 to perform preparation processing. The mobile manipulator 10 then, under control of the local controller 11 receiving the instruction for preparation processing, moves to the position of the first incubator, opens the door of the first incubator, and grips the first container with the hand 13B. The mobile manipulator 10 moves the first container in a gripped state to the workbench, and arranges the first container on the workbench.

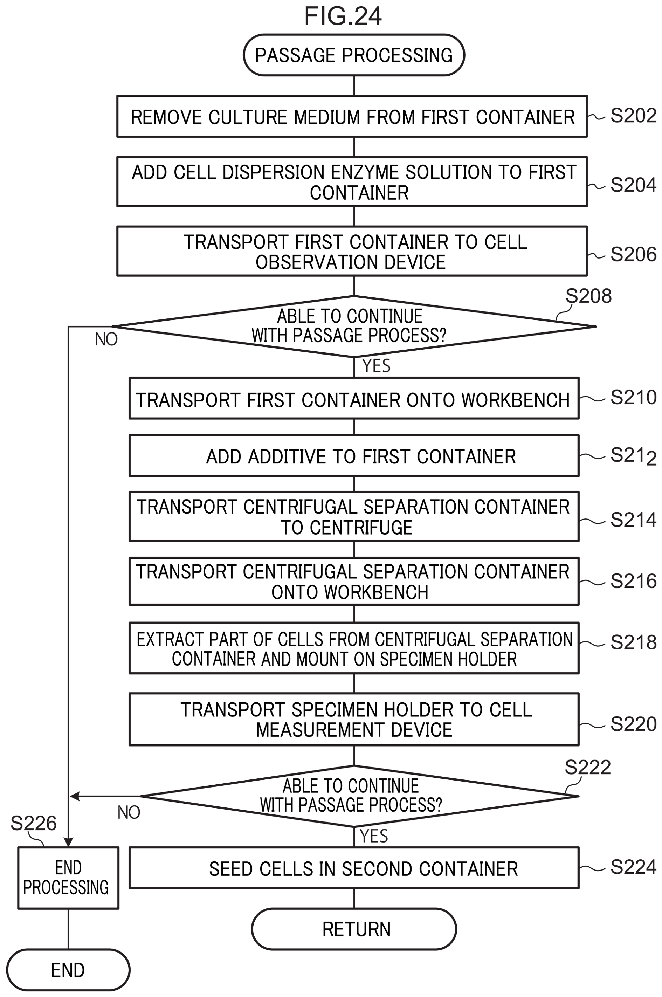

Next, at step S200, the integration controller 30 causes the mobile manipulator 10 and the workbench robot 20 to execute a passage process. Description follows regarding a passage process, with reference to Fig. 24.

At step S202, the integration controller 30 instructs the local controller 21 of the workbench robot 20 to remove a culture medium from the first container. The local controller 21 receiving the instruction then controls the workbench robot 20 so as to, for example, grip the first container with the hand 22BL, grip an aspirator with the hand 22BR, and manipulate the aspirator so as to suction and remove the culture medium inside the first container.

Next, at step S204, the integration controller 30 instructs the local controller 21 of the workbench robot 20 to add a cell dispersion enzyme solution to the first container. The cell dispersion enzyme solution is an example of a cell dispersion reagent and is, for example, trypsin. The local controller 21 receiving the instruction then controls the workbench robot 20 so as to, for example, grip an electronic pipette using the hand 22BR, manipulate the electronic pipette, and add the cell dispersion enzyme solution into the first container. The manipulation of the electronic pipette is performed, for example, by depressing a discharge button using a finger of the hand 22BR (for example, the second finger 22B2R or the third finger 22B3R).

Next, at step S206, the integration controller 30 instructs the local controller 11 of the mobile manipulator 10 so as to transport the first container to a cell observation device. The local controller 11 receiving the instruction then controls the mobile manipulator 10 so as to, for example, transport the first container to which the cell dispersion enzyme solution has been added on the workbench to a cell observation device such an optical microscope or the like to perform enlargement imaging acquisition and image analysis of an observation target object under computer control, and to arrange the first container at a specific position on the cell observation device. A device having a long object distance capable of observing cells through a transparent wall of the first container may be employed as the cell observation device.

Next, at step S208, the integration controller 30 determines whether or not the integration controller 30 is able to continue with passage processes based on observation results from the cell observation device. For example, the integration controller 30 acquires from the cell observation device an image of cells inside the first container or an image analysis result as the observation result, and determines that continuing with the passage process is possible when the cells are sufficiently separated from the container walls and are in a suspended state. Note that observation of the degree of separation of the cells by the cell observation device may be performed automatically using image processing of the cell observation device or the integration controller 30, or may be performed with manual intervention. Processing transitions to step S210 in cases in which continuing with passage processes is possible, and processing transitions to step S226 when continuing is not possible. Note that instead of transitioning to step S226, observation may be re-attempted by the cell observation device after waiting several minutes. Sometimes separation of cells from the container walls can be expected to progress with the passage of time.

At step S210, the integration controller 30 instructs the local controller 11 of the mobile manipulator 10 so as to transport the first container onto the workbench. The local controller 11 receiving the instruction then controls the mobile manipulator 10 so as to take out and grip the first container from the cell observation device, to transport the first container to the workbench, and to arrange the first container on the workbench. The above step S206 to step S210 may be omitted in cases in which sufficient separation of cells from the container walls can be expected without this being confirmed by a cell observation device. Examples of such cases are when past results for culturing of the same cell type under similar conditions indicated separation of cells from the container walls succeeded under similar conditions.

Next at step S212, the integration controller 30 instructs the local controller 21 of the workbench robot 20 so as to add, to the first container, an additive that is at least one from out of an enzyme reaction stopping solution or a new culture medium. The local controller 21 receiving the instruction then, for example, controls the workbench robot 20 so as to grip the first container with the left hand 22BL, to grip an electronic pipette with the hand 22BR, and to add the additive into the first container. In the following, the first container containing a mixture of the cells and the additive, or a third container to which the contents of the first container have been transferred, is called a centrifugal separation container.

Note that in cases in which the mixture of the cells and the additive are to be transferred to the third container, the integration controller 30 instructs the local controller 21 of the workbench robot 20 thereof. The local controller 21 receiving the instruction then, for example, controls the workbench robot 20 so as to manipulate an electronic pipette and suction the mixture from the first container. The manipulation of the electronic pipette is, for example, by depressing a suction button with one of the fingers of the hand 22BR (for example, the second finger 22B2R or the third finger 22B3R). The local controller 21 then controls the workbench robot 20 so as to, for example, switch to holding the centrifugal separation container such as a conical tube with the hand 22BL. Furthermore, the local controller 21 controls the workbench robot 20 so as to manipulate the electronic pipette, and transfer the suctioned mixture over to the centrifugal separation container. The manipulation of the electronic pipette is performed, for example, by depressing a discharge button using a finger of the hand 22BR (for example, the second finger 22B2R of the third finger 22B3R).

Note that in cases in which the first container is to be employed as is as the centrifugal separation container, a conical tube is employed from the start as the first container rather than a square flask.

Next, at step S214, the integration controller 30 instructs the local controller 11 of the mobile manipulator 10 so as to transport the centrifugal separation container to the centrifuge. The local controller 11 receiving the instruction then controls the mobile manipulator 10 so as to transport the centrifugal separation container on the workbench to the centrifuge and to place the centrifugal separation container in a specific position in the centrifuge (for example, a rotation unit thereof). When doing so, if there is a need in the specification of the centrifuge to set the centrifugal separation container in a member for setting centrifugal separation containers, the local controller 11 controls the mobile manipulator 10 so as to, after taking the member out from inside the centrifuge, then return the member to inside the centrifuge. Furthermore, the local controller 11 may control the mobile manipulator 10 so as to depress a start button of the centrifuge and start running of the centrifuge. Note that a run start manipulation of the centrifuge may be performed by the integration controller 30 transmitting a control signal to the centrifuge.

Next, at step S216, the integration controller 30 instructs the local controller 11 of the mobile manipulator 10 so as to transport the centrifugal separation container onto the workbench. The local controller 11 receiving the instruction then controls the mobile manipulator 10 so as to recover the centrifugal separation container from the centrifuge, to grip and transport the centrifugal separation container to the workbench, and to arrange the centrifugal separation container on the workbench.

Next, at step S218, the integration controller 30 instructs the local controller 21 of the workbench robot 20 so as to extract part of the cells from the centrifugal separation container and to mount them on a specimen holder. The specimen holder employed is one matching the specification of a cell measurement device. For example, the specimen holder is a slide glass for cases in which the cell measurement device is an optical microscope. The slide glass may be configured with an indentation for inserting the specimen. Sometimes, depending on the specification of the cell measurement device, some sort of container may be employed as the specimen holder.

The local controller 21 receiving the instruction then, for example, controls the workbench robot 20 so as to manipulate the electronic micro pipette and suction part of the cells from the centrifugal separation container. The local controller 21 then controls the workbench robot 20 so as to manipulate an electronic micro pipette, and discharge the suctioned cells into, for example, an indentation of a specimen holder such as a cell counting plate. The integration controller 30 may be configured so as to cause the workbench robot 20 to execute processing to stain cells of the measurement target before cell measurement.

Note that the electronic micro pipette is an instrument that enables small quantities of cells to be suctioned and discharged. A configuration may be adopted in which ON/OFF control of suctioning and discharging in the electronic micro pipette is performed using by-wire electronic control through a USB cable or the like, such that ON/OFF manipulation is not performed using the hand 22B of the workbench robot 20.

Next, at step S220, the integration controller 30 instructs the local controller 11 of the mobile manipulator 10 so as to transport the specimen holder to the cell measurement device for measuring the number or density of cells. The local controller 11 receiving the instruction controls the mobile manipulator 10 so as to, for example, transport the specimen holder on the workbench to a cell measurement device such as an optical microscope for performing enlargement imaging acquisition and image analysis of the observation target object under computer control, and to set the specimen holder in a setting unit of the cell measurement device. Note that reference here to "setting unit" indicates a setting location where measurement using the cell measurement device can be performed after the specimen holder has been set there. The specimen holder set in the setting unit may be moved to the measurement location using a function of the cell measurement device, with no need to be measurable while still set in the setting unit.

Next, at step S222, the integration controller 30 determines whether or not a passage process is able to continue based on measurement results from the cell measurement device. For example, a passage process is determined to be able to continue in cases in which the integration controller 30 acquires a number or density of cells in the specimen holder as the measurement results from the cell measurement device, and the number or density of cells is a predetermined threshold or above. Note that measurement of the number or density of cells by the cell measurement device may be performed automatically using image processing, or may be performed with manual intervention. Processing transitions to step S224 in cases in which a passage process is able to continue, and processing transitions to step S226 in cases in which a passage process is not able to continue.

At step S224, the integration controller 30 instructs the local controller 21 of the workbench robot 20 to seed cells in one or plural second containers. Note that the second container may be similar to the first container. For expansion passage processes, sometimes the second container is a container the same as the first container but of greater size, and sometimes there is an increase in the number of containers. For a container provided with plural wells (indentations), each of the wells may be considered as being a second container.

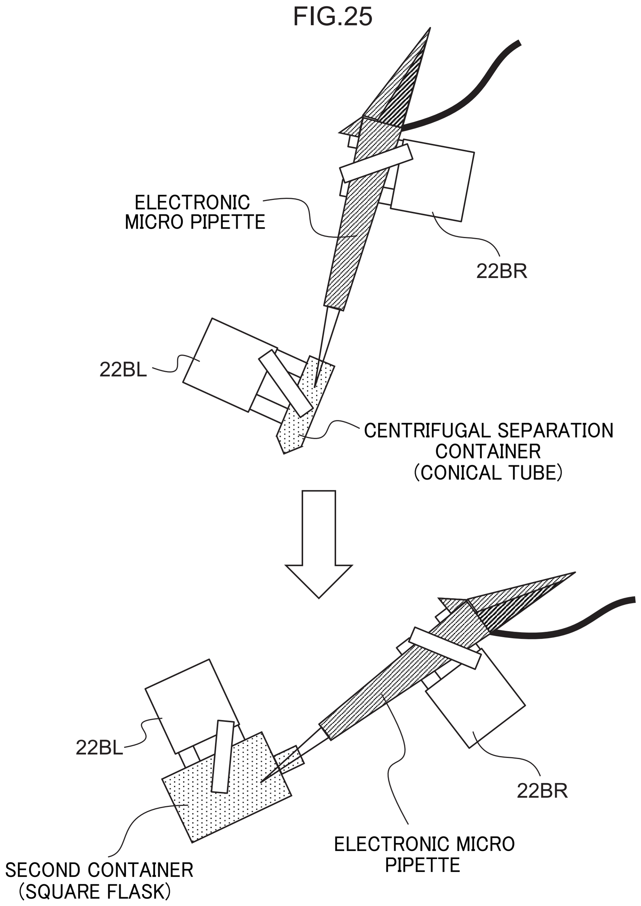

The local controller 21 receiving the instruction controls the workbench robot 20 so as to, for example as illustrated at the top in Fig. 25, manipulate the electronic micro pipette, and to suction cells (including culture medium) of an amount for seeding the second container out from the centrifugal separation container. The local controller 21 then controls the workbench robot 20 so as to, as illustrated at the bottom in Fig. 25, manipulate the electronic micro pipette and to discharge the suctioned cells into the second container that is, for example, a square flask or the like. The local controller 21 repeats such control for the number of second containers. Moreover, as required, an electronic pipette may be employed so as to augment the culture medium in the second containers.

At step S226, the integration controller 30 outputs from the output device 45 that a passage process is not able to continue, and ends the passage process and control processing. When doing so information indicating a reason that the passage process is not able to continue may be included in this output. Adopting such a configuration enables a person, or higher-level system, receiving the output to easily ascertain passage process conditions and take appropriate measures. Note that a configuration may be adopted so as to continue control processing with the next first container inside the first incubator as the target for a passage process. In such cases processing may return to step S100 of the control processing after step S226.

Next, at step S300, the integration controller 30 causes the mobile manipulator 10 to store the second containers holding post-passage process cells in a second incubator as clearing away processing for the passage process. The second incubator may be the same as the first incubator.

Specifically, the integration controller 30 instructs clearing away processing to the local controller 11 of the mobile manipulator 10. Under control of the local controller 11 receiving the instruction of clearing away processing, the mobile manipulator 10 grips the second container on the workbench, moves it to the position of the second incubator, opens the door of the second incubator, and stores the second container inside the second incubator. The control processing is then ended.

As described above, the cell culture system according to the second exemplary embodiment enables culturing of cells by a robot system to be automated based on a plan for culturing cells of a required amount as created by a cell culture plan creation device.

Third Exemplary Embodiment

Similarly to in the second exemplary embodiment, the third exemplary embodiment will also be described for a case in which processing of cell culturing is executed by a robot system according to a cell culture plan created by the cell culture plan creation device. Note that in the third exemplary embodiment, the same reference numerals will be appended across the drawings to the same parts as in the first and second exemplary embodiments, and detailed explanation thereof will be omitted.

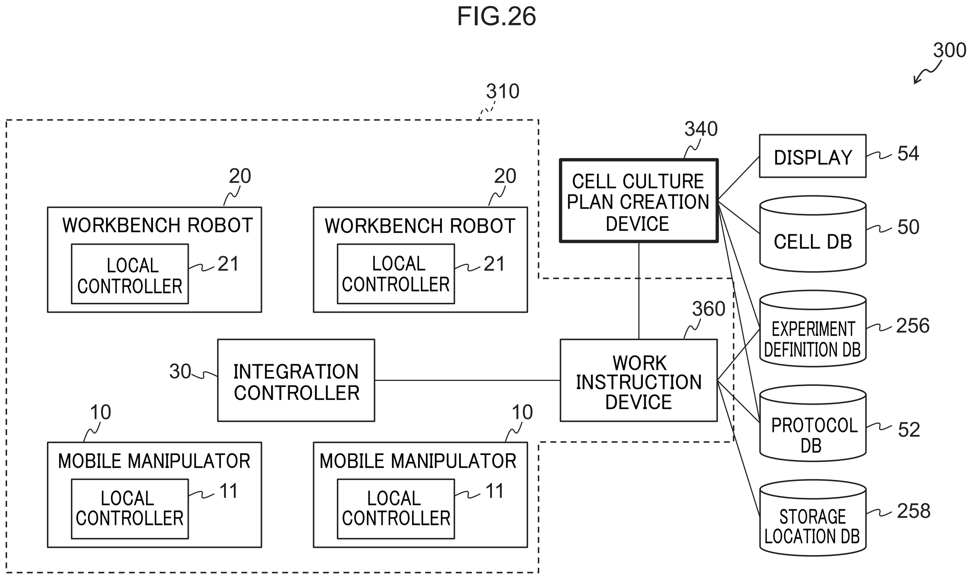

As illustrated in Fig. 26, a cell culture system 300 according to a third exemplary embodiment includes a cell culture plan creation device 340 and a robot system 310.

The cell culture plan creation device 340 is connected to a cell DB 50, a protocol DB 52, an experiment definition DB 256, and a display 54.

The robot system 310 includes mobile manipulators 10, workbench robots 20, an integration controller 30, and a work instruction device 360. The work instruction device 360 is connected to each of the protocol DB 52, the experiment definition DB 256, and a storage location DB 258. The protocol DB 52 and the experiment definition DB 256 are shared with the cell culture plan creation device 340.

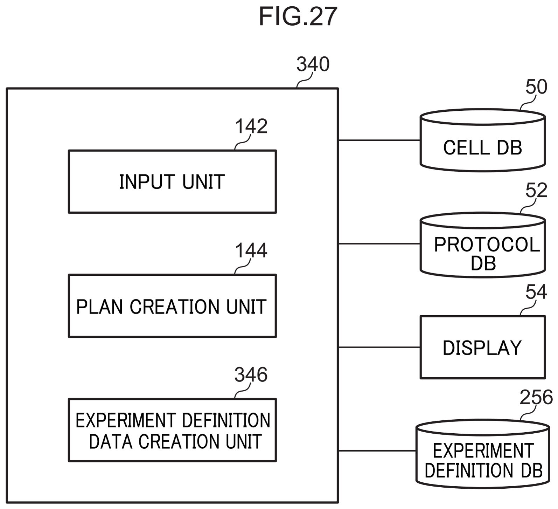

As illustrated in Fig. 27, the cell culture plan creation device 340 includes, as functional configuration, an input unit 142, a plan creation unit 144, and an experiment definition data creation unit 346. Each functional configuration is implemented by the CPU 41 reading a cell culture plan creation program stored on the storage device 43 and expanding and executing the cell culture plan creation program in the memory 42.

The experiment definition data creation unit 346, similarly to the experiment definition data creation unit 262 of the work instruction device 260 in the second exemplary embodiment, creates experiment definition data for each process based on the cell culture plan created by the plan creation unit 144 and stores this in the experiment definition DB 256.

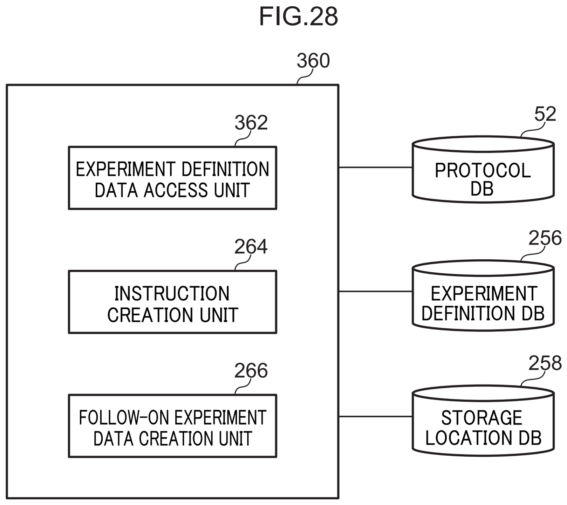

Fig. 28 is a block diagram illustrating an example of a functional configuration of the work instruction device 360. As illustrated in Fig. 28, the work instruction device 360 includes, as functional configuration, an experiment definition data access unit 362, an instruction creation unit 264, and a follow-on experiment data creation 266. Each of the functional configuration is implemented by a CPU reading a work instruction program stored on a storage device and expanding and executing the work instruction program in the memory.

The experiment definition data access section 362 accesses the experiment definition DB 256, acquires the experiment definition data for each process stored in the experiment definition DB 256, and passes this across to the instruction creation unit 264. The physical location of the experiment definition DB 256 may be freely selected as long as it is accessible by the work instruction device 360. For example, the experiment definition DB 256 may be stored in the storage device 43 inside the work instruction device 360, may be stored in an external storage device connected to the work instruction device 360, or may be stored on a server accessible over a network. For example, an indirect access format may be employed in which the work instruction device 360 designates and requests experiment definition data from an external device identified by data configured by a key identifying an experiment such as an experiment ID such that, in response to this request, the instructed experiment definition data is sent to the work instruction device 360.

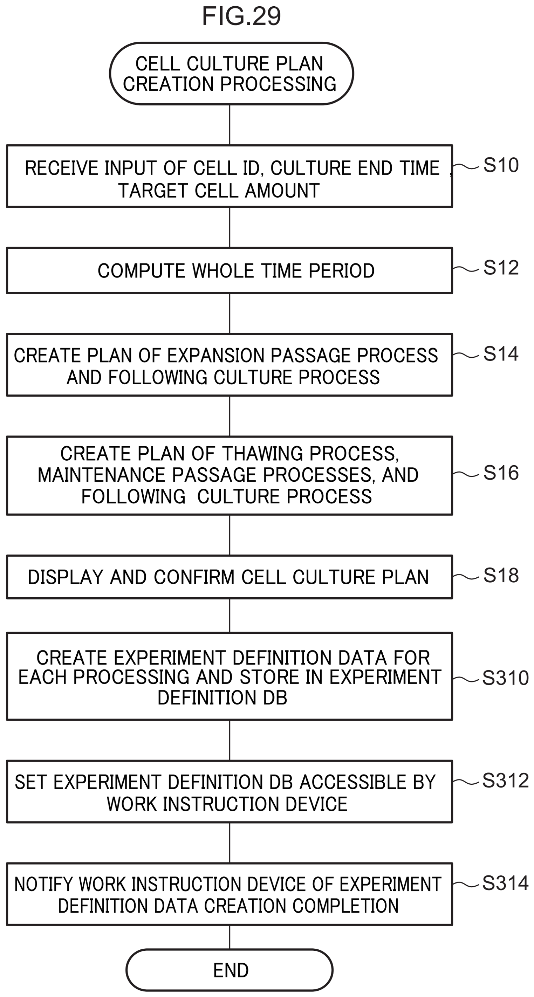

Next, description follows regarding operation of the cell culture system 300 according to the third exemplary embodiment. Fig. 29 is a flowchart illustrating a flow of cell culture plan creation processing executed by the CPU 41 of the cell culture plan creation device 340.

After passing through steps S10 to S18 similar to those of the cell culture plan creation processing according to the first exemplary embodiment (Fig. 5), at the next step S310, the experiment definition data creation unit 346 creates experiment definition data for each process and stores this in the experiment definition B256. Next, at step S312, the experiment definition data creation unit 346 performs setting such that the experiment definition DB 256 is accessible by the work instruction device 360. Next, at step S314, the experiment definition data creation unit 346 notifies the work instruction device 360 of experiment definition data creation and experiment definition DB 256 storage completion, and then ends the cell culture plan creation processing.

The work instruction device 360 executes the work instruction processing illustrated in Fig. 22. Note that in the third exemplary embodiment, at step S20 instead of creating the experiment definition data, the experiment definition data access unit 362 acquires the experiment definition data of each process by accessing the experiment definition DB 256, and passes this across to the instruction creation unit 264.

As described above, the cell culture system according to the third exemplary embodiment enables automation of cell culturing by a robot system based on a plan for culturing cells at a required amount as created by the cell culture plan creation device.

Note that the third exemplary embodiment may be configured such that the experiment definition data created by the experiment definition data creation unit of the cell culture plan creation device does not include all of the experiment definition data, with the work instruction device augmenting the lacking portions of the experiment definition data. For example, the cell culture plan creation device may create experiment definition data that includes the first most preparation transport information (container ID, storage location ID) but does not include subsequent preparation transport information, with the work instruction device completing the experiment definition data by adding the subsequent preparation transport information thereto.

Moreover, in the second exemplary embodiment and the third exemplary embodiment, the cell culture plan creation device and the work instruction device may be configured by functional parts inside the same computer. Furthermore, the integration controller may be configured as a functional part inside the same computer.

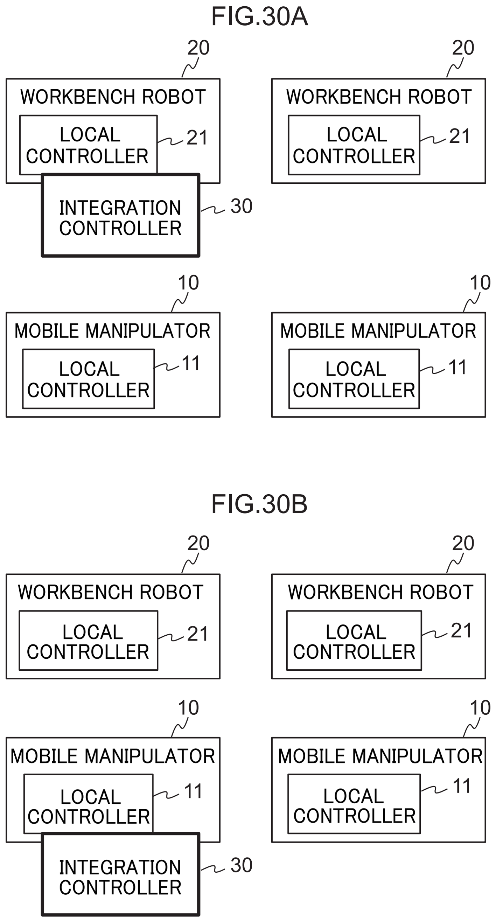

Moreover, although in the second exemplary embodiment and the third exemplary embodiment cases have been described which employ, as examples of controllers, the integration controller 30 independent to the mobile manipulator 10 and the workbench robot 20, as illustrated in Fig. 14, there is no limitation thereto. For example, as illustrated in Fig. 30A, an integration controller 30 may be installed to one of the workbench robots 20 or, as illustrated in Fig. 30B, an integration controller 30 may be installed to one of the mobile manipulators 10. In such cases the work instruction device may be configured capable of wireless communication with the integration controller 30.

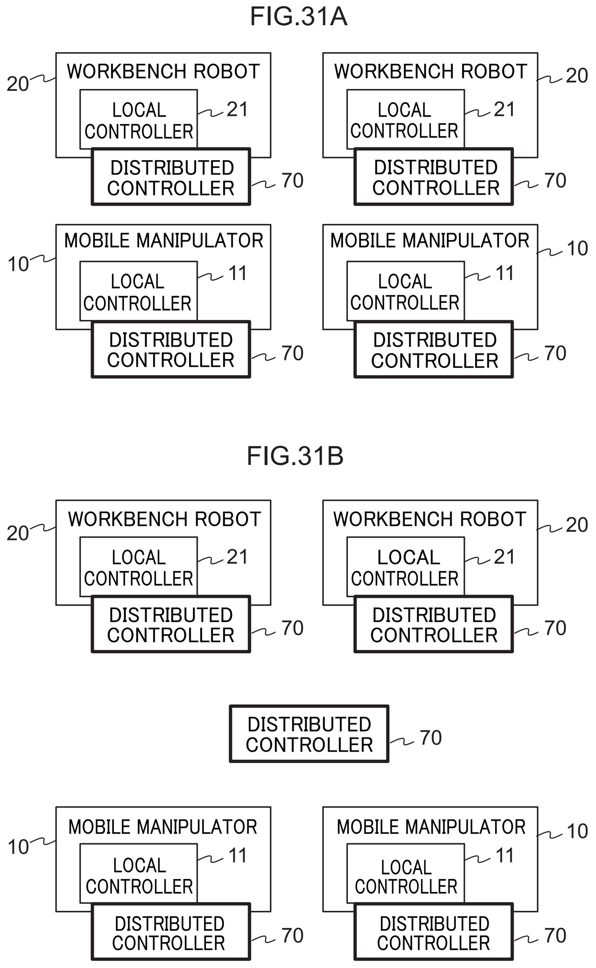

Moreover, as illustrated in Fig. 31A and Fig. 31B, the controller may be configured by plural distributed controllers 70. In such cases, the distributed controllers 70 communicate with each other, and control such that the mobile manipulators 10 and the workbench robots 20 operate in coordination with each other. In such cases, the work instruction device may be configured capable of wireless communication with each of the distributed controllers 70.

Note that a configuration may be adopted in which the local controllers 11, 21 are not respectively provided to the mobile manipulators 10 and the workbench robots 20, and the integration controller 30 or the distributed controllers 70 control each operation of each of the motors of the mobile manipulators 10 and the workbench robots 20.

Moreover, although description has been given in the above exemplary embodiments of examples in which the experiment definition DB is a database having a data structure in which a data record is formed for each experiment ID, there is no limitation thereto. For example, as long as a database is formed such that necessary data for performing an instructed experiment can be extracted in a format such as a relational database, the data structure of the experiment definition data is not important.

Moreover, the cell culture plan created by the cell culture plan creation device is not limited to application to automatic execution of cell culturing by a robot system as in the second exemplary embodiment and the third exemplary embodiment. For example, application may be made to execution of cell culture manually based on the created cell culture plan. Moreover, there is no limitation to an objective of actual execution of cell culturing according to the created cell culture plan, and the plan may be employed for the objective of finding a better plan by repeatedly trying plans.

Moreover, the robot system processing executed by the CPU reading in software (a program) in the above exemplary embodiment may be executed by various processors other than a CPU. Examples of such processors include programmable logic devices (PLD) that allow circuit configuration to be modified post-manufacture, such as a field-programmable gate array (FPGA), and dedicated electric circuits and the like, these being processors including a circuit configuration custom-designed to execute specific processing, such as an application specific integrated circuit (ASIC). The robot system processing may be executed by any one of these various types of processors, or may be executed by a combination of two or more of the same type or different type of processor (such as plural FPGAs, or a combination of a CPU and an FPGA). The hardware structure of these various types of processors is more specifically an electric circuit combining circuit elements such as semiconductor elements.

Moreover, although embodiments were described in the above exemplary embodiments in which programs were pre-stored (installed) on storage devices, there is no limitation thereto. The programs may be provided in a format stored on a storage medium such as a CD-ROM, DVD-ROM, Blu-ray disc, USB memory, or the like. Moreover, the programs may be provided in a format downloadable from an external device over a network.

The following Supplements related to the present disclosure are disclosed.

Supplement 1

A cell culture plan creation device (40, 340) including:

an input unit (142) that receives input of a cell culture end time and a target cell amount at the culture end time; and
a plan creation unit (144) that creates a cell culture plan including a first execution instruction for one or more passage process sequentially executed so as to obtain the target cell amount at the culture end time, and a second execution instruction for a culture process executed following each of the passage processes, wherein in the cell culture plan the first execution instruction includes information to identify a type of the passage process and information to identify a timing to execute the passage process.

Supplement 2

The cell culture plan creation device of Supplement 1, wherein:

the cell culture plan further includes a third execution instruction for a thawing process of the cells executed in advance of a first most of the passage processes, and a fourth execution instruction for an initial culture process of the cells that have been thawed; and
the third execution instruction including information to identify a timing to execute the thawing process.

Supplement 3

The cell culture plan creation device of Supplement 1 or Supplement 2, wherein the type of the passage process identified in the first execution instruction includes at least one out of a type of passage process belonging to maintenance passage processes or a type of passage process belonging to expansion passage processes.

Supplement 4

The cell culture plan creation device of Supplement 3, wherein, in cases in which both the type of the passage process belonging to maintenance passage processes and the type of the passage process belonging to expansion passage processes are identified in the first execution instruction, the plan creation unit identifies a timing to execute each of the passage processes such that the timing to execute the passage process belonging to expansion passage processes is later than the timing to execute the passage process belonging to maintenance passage processes.

Supplement 5

The cell culture plan creation device of any one of Supplement 1 to Supplement 4, wherein the plan creation unit also outputs, as a signal to display on a display, the created cell culture plan with first graphics representing the passage processes and second graphics representing the culture processes arranged along a first direction according to process order in which each of the passage processes and the culture processes is executed.

Supplement 6

The cell culture plan creation device of Supplement 5, wherein the second graphics are graphics that have a size in a second direction orthogonal to the first direction that increases according to elapse of time in the first direction.

Supplement 7

A cell culture system (200, 300) including:

the cell culture plan creation device (40, 340) of any one of Supplement 1 to Supplement 6; and
a robot system (210, 310) that includes a mobile manipulator equipped with a mechanism to move over a floor and a mechanism to grip an object and a workbench robot that is fixed to a workbench and is equipped with a mechanism to grip an object on the workbench, and that executes a cell culture experiment according to the cell culture plan.

Explanation of the Reference Numerals

200, 300 cell culture system
210, 310 robot system
10 mobile manipulator
11 local controller
12 trolley
12A drive wheel
13 robot arm
13A arm
13B hand
20 workbench robot
21 local controller
22 robot arm
22A arm
22B, 22BL, 22BR hand
22B1, 22B1L, 22B1R first finger
22B2, 22B2L, 22B2R second finger
22B3, 22B3L, 22B3R third finger
23 vision sensor
30 integration controller
40, 340 cell culture plan creation device
41 CPU
42 memory
43 storage device
44 input device
45 output device

46 storage medium reading device
47 communication I/F
48 bus
142 input unit
144 plan creation unit
346 experiment definition data creation unit
50 cell DB
52 protocol DB
54 display
256 experiment definition DB
258 storage location DB
260, 360 work instruction device
262 experiment definition data creation unit
264 instruction creation unit
266 follow-on experiment data creation unit
362 experiment definition data access unit
70 distributed controller

**Claims**

1. A cell culture plan creation device, comprising:

   an input unit that receives input of a cell culture end time and a target cell amount at the culture end time; and
   a plan creation unit that creates a cell culture plan including a first execution instruction for one or more passage processes sequentially executed so as to obtain the target cell amount at the culture end time, and a second execution instruction for a culture process executed following each passage process, wherein, in the cell culture plan, the first execution instruction includes information to identify a type of the passage process and information to identify a timing to execute the passage process.

2. The cell culture plan creation device of claim 1, wherein:

   the cell culture plan further includes a third execution instruction for a thawing process of the cells, which is executed in advance of a first most of the passage processes, and a fourth execution instruction for an initial culture process of the cells that have been thawed; and
   the third execution instruction includes information to identify a timing to execute the thawing process.

3. The cell culture plan creation device of claim 1 or claim 2, wherein the type of the passage process identified in the first execution instruction includes at least one of a type of passage process belonging to maintenance passage processes or a type of passage process belonging to expansion passage processes.

4. The cell culture plan creation device of claim 3, wherein, in a case in which both the type of the passage process belonging to maintenance passage processes and the type of the passage process belonging to expansion passage processes are identified in the first execution instruction, the plan creation unit identifies a timing to execute each passage process such that a timing to execute the passage process belonging to expansion passage processes is later than a timing to execute the passage process belonging to maintenance passage processes.

5. The cell culture plan creation device of claim 1 or claim 2, wherein the plan creation unit outputs, as a signal to display on a display, the created cell culture plan with first graphics representing the passage processes and second graphics representing the culture processes arranged along a first direction according to a process order in which each passage process and each culture process is executed.

6. The cell culture plan creation device of claim 5, wherein the second graphics are graphics that have a size in a second direction, orthogonal to the first direction, which increases according to elapse of time in the first direction.

7. A cell culture system, comprising:

   a cell culture plan creation device including an input unit that receives input of a cell culture end time and a target cell amount at the culture end time, and a plan creation unit that creates a cell culture plan including a first execution instruction for one or more passage processes sequentially executed so as to obtain the target cell amount at the culture end time and a second execution instruction for a culture process executed following each passage process, wherein, in the cell culture plan, the first execution instruction includes information to identify a type of the passage process and information to identify a timing to execute the passage process; and

a robot system that includes a mobile manipulator equipped with a mechanism to move over a floor and a mechanism to grip an object and a workbench robot that is fixed to a workbench and is equipped with a mechanism to grip an object on the workbench, and that executes a cell culture experiment according to the cell culture plan.

FIG.1

40

44

INPUT DEVICE

45

OUTPUT
DEVICE

46

STORAGE MEDIUM
READING DEVICE

47

COMMUNICATION
I/F

41

CPU

42

MEMORY

43

STORAGE
DEVICE

48

FIG.2

FIG.3

50

| CELL ID | MULTIPLICATION RATE $\mu$ (MULTIPLES/DAY) | THAWING PROCESS PROTOCOL ID | MAINTENANCE PASSAGE PROCESS PROTOCOL ID | EXPANSION PASSAGE PROCESS PROTOCOL ID |
|---|---|---|---|---|
| CEL-012-04 | 16 | PRT-01-17 | PRT-02-23 | PRT-03-35 PRT-03-36 PRT-03-37 |
| ... | | | | |

# FIG.4

PROTOCOL ID: PRT-02-23

PROCESS TYPE: MAINTENANCE PASSAGE

TARGET CELLS: CEL-012-04

PROCESS PROCEDURE:

1. REMOVE OLD CULTURE MEDIUM

2. ADD CELL DISPERSION ENZYME SOLUTION

3. CHECK CELL SEPARATION STATE WITH CELL OBSERVATION DEVICE

4. ADD NEW CULTURE MEDIUM AND SUSPEND IF SEPARATION SUCCEEDS

5. CENTRIFUGE

6. REMOVE SUPERNATE AND SUSPEND

7. EXTRACT SMALL AMOUNT OF CELLS

8. MEASURE DENSITY OF EXTRACTED CELLS WITH CELL MEASUREMENT DEVICE

9. SEED CELLS IN NEW CULTURE MEDIUM WHEN CELL DENSITY IN NORMAL RANGE

# FIG.5

CELL CULTURE PLAN
CREATION PROCESSING

RECEIVE INPUT OF CELL ID, CULTURE
END TIME, TARGET CELL AMOUNT — S10

COMPUTE WHOLE TIME PERIOD — S12

CREATE PLAN OF EXPANSION PASSAGE
PROCESS AND FOLLOWING CULTURE
PROCESS — S14

CREATE PLAN OF THAWING PROCESS,
MAINTENANCE PASSAGE PROCESSES,
AND FOLLOWING CULTURE PROCESS — S16

DISPLAY AND CONFIRM CELL CULTURE PLAN — S18

END

# FIG.6

| | INITIAL CULTURE | FIRST TIME PASSAGE PROCESS | CULTURING | SECOND TIME PASSAGE PROCESS | CULTURING | THIRD TIME PASSAGE PROCESS | CULTURING | FOURTH TIME PASSAGE PROCESS | CULTURING | TOTAL TIME PERIOD |
|---|---|---|---|---|---|---|---|---|---|---|
| CULTURE TIME PERIOD (DAYS) | 3 | | 3 | | 3 | | 3 | | 3 | 15 |
| POST-CULTURE CELL AMOUNT (UNITS) | 4.1 | | 4.1 | | 4.1 | | 8.2 | | 16.4 | |
| USABLE CELL AMOUNT (UNITS) | 2 | | 2 | | 2 | | 4 | | 8 | |
| SEEDING CONTAINER SIZE | | SMALL | | SMALL | | SMALL | | SMALL | | |
| SEEDING CONTAINER NUMBER | | 1 | | 1 | | 2 | | 4 | | |
| SEEDING CELL AMOUNT PER SINGLE CONTAINER (UNITS) | | 1 | | 1 | | 1 | | 1 | | |
| TOTAL SEEDING CELL AMOUNT (UNITS) | | 1 | | 1 | | 2 | | 4 | | |
| PASSAGE TYPE | | MAINTENANCE | | MAINTENANCE | | EXPANSION | | EXPANSION | | |

EP 4 722 329 A1

# FIG.7

CELL ID: CEL-012-04  STRAIN – 14
CULTURE END TIME: 22 FEB 2023 10:00
TARGET CELL AMOUNT: 8 UNITS

CELL AMOUNT

THAWING

INITIAL CULTURING

MAINTENANCE PASSAGE

CULTURING

MAINTENANCE PASSAGE

CULTURING

EXPANSION PASSAGE

CULTURING

EXPANSION PASSAGE

CULTURING — 2 UNITS

CULTURING — 2 UNITS

CULTURING — 2 UNITS

CULTURING — 2 UNITS

"SMALL" CONTAINER

"SMALL" CONTAINER

"SMALL" CONTAINER

"SMALL" CONTAINER

"SMALL" CONTAINER

2023/2/07 10:00

2023/2/10 10:00

2023/2/13 10:00

2023/2/16 10:00

2023/2/19 10:00

2023/2/22 10:00

EP 4 722 329 A1

# FIG.8

CASE OF 3 DAYS CULTURING

MAINTENANCE PASSAGE

MAINTENANCE PASSAGE

CULTURING

EXPONENTIAL FUNCTION PATTERN CURVE

CASE OF 4 DAYS CULTURING

MAINTENANCE PASSAGE

MAINTENANCE PASSAGE

CULTURING

EXPONENTIAL FUNCTION PATTERN CURVE

## FIG.9

| | INITIAL CULTURE | FIRST TIME PASSAGE PROCESS | CULTURING | SECOND TIME PASSAGE PROCESS | CULTURING | TOTAL TIME PERIOD |
|---|---|---|---|---|---|---|
| CULTURE TIME PERIOD (DAYS | 2 | | 4 | | 4 | 10 |
| POST-CULTURE CELL AMOUNT (UNITS) | 2.56 | | 6.6 | | 19.8 | |
| USABLE CELL AMOUNT (UNITS) | 1 | | 3 | | 9 | |
| SEEDING CONTAINER SIZE | | MEDIUM | | MEDIUM | | |
| SEEDING CONTAINER NUMBER | | 1 | | 3 | | |
| SEEDING CELL AMOUNT PER SINGLE CONTAINER (UNITS) | | 1 | | 1 | | |
| TOTAL SEEDING CELL AMOUNT (UNITS) | | 1 | | 3 | | |
| PASSAGE TYPE | | MAINTENANCE | | EXPANSION | | |

# FIG.10

CELL ID: CEL-012-04  STRAIN - 14
CULTURE END TIME: 22 FEB 2023 10:00
TARGET CELL AMOUNT:  8 UNITS

CELL AMOUNT

EXPANSION PASSAGE

THAWING

MAINTENANCE PASSAGE

INITIAL CULTURING

CULTURING    3 UNITS

CULTURING

CULTURING    3 UNITS

CULTURING    3 UNITS

"SMALL" CONTAINER

"MEDIUM" CONTAINER

"MEDIUM" CONTAINER

2023/2/12
10:00

2023/2/14
10:00

2023/2/18
10:00

2023/2/22
10:00

EP 4 722 329 A1

# FIG.11

| | INITIAL CULTURE | FIRST TIME PASSAGE PROCESS | CULTURING | SECOND TIME PASSAGE PROCESS | CULTURING | THIRD TIME PASSAGE PROCESS | CULTURING | FOURTH TIME PASSAGE PROCESS | CULTURING | TOTAL TIME PERIOD |
|---|---|---|---|---|---|---|---|---|---|---|
| CULTURE TIME PERIOD (DAYS) | 3 | | 3 | | 3 | | 3 | | 3 | 15 |
| POST-CULTURE CELL AMOUNT (UNITS) | 4.1 | | 4.1 | | 4.1 | | 8.2 | | 16.4 | |
| USABLE CELL AMOUNT (UNITS) | 2 | | 2 | | 2 | | 4 | | 8 | |
| SEEDING CONTAINER SIZE | | SMALL | | SMALL | | LARGE | | LARGE | | |
| SEEDING CONTAINER NUMBER | | 1 | | 1 | | 1 | | 2 | | |
| SEEDING CELL AMOUNT PER SINGLE CONTAINER (UNITS) | | 1 | | 1 | | 2 | | 2 | | |
| TOTAL SEEDING CELL AMOUNT (UNITS) | | 1 | | 1 | | 2 | | 4 | | |
| PASSAGE TYPE | | MAINTENANCE | | MAINTENANCE | | EXPANSION | | EXPANSION | | |

EP 4 722 329 A1

# FIG.12

CELL ID: CEL-012-04  STRAIN - 14
CULTURE END TIME: 22 FEB 2023 10:00
TARGET CELL AMOUNT: 8 UNITS

CELL AMOUNT

THAWING — INITIAL CULTURING

MAINTENANCE PASSAGE — CULTURING

MAINTENANCE PASSAGE — CULTURING

EXPANSION PASSAGE — CULTURING

EXPANSION PASSAGE — CULTURING — 4 UNITS

CULTURING — 4 UNITS

"SMALL" CONTAINER

"SMALL" CONTAINER

"SMALL" CONTAINER

"LARGE" CONTAINER

"LARGE" CONTAINER

2023/2/07 10:00

2023/2/10 10:00

2023/2/13 10:00

2023/2/16 10:00

2023/2/19 10:00

2023/2/22 10:00

EP 4 722 329 A1

# FIG.13

FIG.14

# FIG.15

# FIG.16

WORKBENCH

FIG.17

# FIG.18

THAWING PROCESS EXPERIMENT DEFINITION DATA

| EXPERIMENT ID | START DATE/TIME | CELL ID | PRE-EXPERIMENT CELL ID CLASSIFICATION NUMBER | POST-EXPERIMENT CELL ID CLASSIFICATION NUMBER | PRE-EXPERIMENT PASSAGE COMPLETE OCCURRENCE NUMBER | POST-EXPERIMENT CULTURE TIME | PROTOCOL ID | PRE-EXPERIMENT CONTAINER ID | PRE-EXPERIMENT CONTAINER STORAGE LOCATION ID | POST-EXPERIMENT CONTAINER ID | POST-EXPERIMENT CONTAINER STORAGE LOCATION ID |
|---|---|---|---|---|---|---|---|---|---|---|---|
| 00156 | 2023/2/7 10:00 | CEL-012-04 | -14 | -14 | 0 | 71h | PRT-01-17 | CTN-01-07-00436 | PLA-01-01-02-05-01 | CTN-02-11-0294 | PLA-02-05-03-04-01 |

## FIG.19

MAINTENANCE PASSAGE PROCESS EXPERIMENT DEFINITION DATA

| EXPERIMENT ID | START DATE/TIME | CELL ID | PRE- EXPERIMENT CELL ID CLASSIFICATION NUMBER | POST- EXPERIMENT CELL ID CLASSIFICATION NUMBER | PRE- EXPERIMENT PASSAGE COMPLETE OCCURRENCE NUMBER | POST- EXPERIMENT CULTURE TIME | PROTOCOL ID | PRE- EXPERIMENT CONTAINER ID | PRE- EXPERIMENT CONTAINER STORAGE LOCATION ID | POST- EXPERIMENT CONTAINER ID | POST- EXPERIMENT CONTAINER STORAGE LOCATION ID |
|---|---|---|---|---|---|---|---|---|---|---|---|
| 00181 | 2023/2/10 10:00 | CEL-012-04 | -14 | -14 | 0 | 71h | PRT-02-23 | CTN-02-11-0294 | PLA-02-05-03-04-01 | CTN-02-11-0301 | PLA-02-05-03-04-01 |
| 00186 | 2023/2/13 10:00 | CEL-012-04 | -14 | -14 | 1 | 71h | PRT-02-23 | CTN-02-11-0301 | PLA-02-05-03-04-01 | CTN-02-11-0307 | PLA-02-05-03-04-01 |

# FIG.20

EXPANSION PASSAGE PROCESS EXPERIMENT DEFINITION DATA

| EXPERIMENT ID | START DATE/TIME | CELL ID | PRE-EXPERIMENT CELL ID CLASSIFICATION NUMBER | POST-EXPERIMENT CELL ID CLASSIFICATION NUMBER | PRE-EXPERIMENT PASSAGE COMPLETE OCCURRENCE NUMBER | POST-EXPERIMENT CULTURE TIME | PROTOCOL ID | PRE-EXPERIMENT CONTAINER ID | PRE-EXPERIMENT CONTAINER STORAGE LOCATION ID | POST-EXPERIMENT CONTAINER ID | POST-EXPERIMENT CONTAINER STORAGE LOCATION ID |
|---|---|---|---|---|---|---|---|---|---|---|---|
| 00193 | 2023/2/16 10:00 | CEL-012-04 | -14 | -14-1; -14-2 | 2 | 71h | PRT-03-35 | CTN-02-11-0307 | PLA-02-05-03-04-01 | CTN-02-11-0312; CTN-02-11-0313; | PLA-02-05-02-01-01; PLA-02-05-02-01-02 |
| 00202 | 2023/2/19 10:00 | CEL-012-04 | -14-1 | -14-1-1; -14-1-2 | 3 | 71h | PRT-03-35 | CTN-02-11-0312 | PLA-02-05-02-01-01 | CTN-02-11-0314; CTN-02-11-0315; | PLA-02-05-02-03-02-01; PLA-02-05-03-02-02 |
| 00203 | 2023/2/19 10:00 | CEL-012-04 | -14-2 | -14-2-1; -14-2-2 | 3 | 71h | PRT-03-35 | CTN-02-11-0313 | PLA-02-05-02-01-02 | CTN-02-11-0316; CTN-02-11-0317; | PLA-02-05-03-03-02-03; PLA-02-05-03-02-04 |

EP 4 722 329 A1

# FIG.21

| EXPERIMENT ID | START DATE/TIME | CELL ID | PRE-EXPERIMENT CELL ID CLASSIFICATION NUMBER | POST-EXPERIMENT CELL ID CLASSIFICATION NUMBER | PRE-EXPERIMENT PASSAGE COMPLETE OCCURRENCE NUMBER | POST-EXPERIMENT CULTURE TIME | PROTOCOL ID | PRE-EXPERIMENT CONTAINER ID | PRE-EXPERIMENT CONTAINER STORAGE LOCATION ID | POST-EXPERIMENT CONTAINER ID | POST-EXPERIMENT CONTAINER STORAGE LOCATION ID |
|---|---|---|---|---|---|---|---|---|---|---|---|
| | | CEL-012-04 | -14-1-1 | | 4 | | | CTN-02-11-0314 | PLA-02-05-03-02-01 | | |
| | | CEL-012-04 | -14-1-2 | | 4 | | | CTN-02-11-0315 | PLA-02-05-03-02-02 | | |
| | | CEL-012-04 | -14-2-1 | | 4 | | | CTN-02-11-0316 | PLA-02-05-03-02-03 | | |
| | | CEL-012-04 | -14-2-2 | | 4 | | | CTN-02-11-0317 | PLA-02-05-03-02-04 | | |

EP 4 722 329 A1

# FIG.22

```
          ┌─────────────────────────────────┐
          │  WORK INSTRUCTION PROCESSING    │
          └─────────────────────────────────┘
                          │
  ┌──────────────────────────────────────────────┐
  │ CREATE EXPERIMENT DEFINITION DATA FOR        │ ─ S20
  │ EACH PROCESS AND STORE  IN EXPERIMENT        │
  │ DEFINITION DB                                │
  └──────────────────────────────────────────────┘
                          │
  ┌──────────────────────────────────────────────┐
  │ INSTRUCT INTEGRATION CONTROLLER TO           │ ─ S22
  │ EXECUTE EXPERIMENT                           │
  └──────────────────────────────────────────────┘
                          │
  ┌──────────────────────────────────────────────┐
  │ CREATE FOLLOW-ON EXPERIMENT DATA AND         │ ─ S24
  │ STORE IN EXPERIMENT  DEFINITION DB           │
  └──────────────────────────────────────────────┘
                          │
          ┌─────────────────────────────────┐
          │             END                 │
          └─────────────────────────────────┘
```

# FIG.23

CONTROL PROCESSING

PREPARATION PROCESSING — S100

PASSAGE PROCESSING — S200

CLEARING AWAY PROCESSING — S300

END

## FIG.24

PASSAGE PROCESSING

REMOVE CULTURE MEDIUM FROM FIRST CONTAINER — S202

ADD CELL DISPERSION ENZYME SOLUTION TO FIRST CONTAINER — S204

TRANSPORT FIRST CONTAINER TO CELL OBSERVATION DEVICE — S206

S208
ABLE TO CONTINUE WITH PASSAGE PROCESS?

NO

YES

TRANSPORT FIRST CONTAINER ONTO WORKBENCH — S210

ADD ADDITIVE TO FIRST CONTAINER — S212

TRANSPORT CENTRIFUGAL SEPARATION CONTAINER TO CENTRIFUGE — S214

TRANSPORT CENTRIFUGAL SEPARATION CONTAINER ONTO WORKBENCH — S216

EXTRACT PART OF CELLS FROM CENTRIFUGAL SEPARATION CONTAINER AND MOUNT ON SPECIMEN HOLDER — S218

TRANSPORT SPECIMEN HOLDER TO CELL MEASUREMENT DEVICE — S220

S222
ABLE TO CONTINUE WITH PASSAGE PROCESS?

NO

YES

S226
END PROCESSING

SEED CELLS IN SECOND CONTAINER — S224

RETURN

END

# FIG.25

ELECTRONIC
MICRO PIPETTE

22BR

22BL

CENTRIFUGAL SEPARATION
CONTAINER
(CONICAL TUBE)

22BL

SECOND CONTAINER
(SQUARE FLASK)

ELECTRONIC MICRO
PIPETTE

22BR

# FIG.26

EP 4 722 329 A1

# FIG.27

340

142
INPUT UNIT

144
PLAN CREATION UNIT

346
EXPERIMENT DEFINITION
DATA CREATION UNIT

50
CELL DB

52
PROTOCOL
DB

54
DISPLAY

256
EXPERIMENT
DEFINITION DB

# FIG.28

360

362

| EXPERIMENT DEFINITION DATA ACCESS UNIT |

52

| PROTOCOL DB |

264

| INSTRUCTION CREATION UNIT |

256

| EXPERIMENT DEFINITION DB |

266

| FOLLOW-ON EXPERIMENT DATA CREATION UNIT |

258

| STORAGE LOCATION DB |

# FIG.29

```
┌─────────────────────────┐
│    CELL CULTURE PLAN     │
│   CREATION PROCESSING    │
└─────────────────────────┘
             │
┌─────────────────────────────────────────────┐
│ RECEIVE INPUT OF CELL ID, CULTURE END TIME , │  ～S10
│           TARGET CELL AMOUNT                 │
└─────────────────────────────────────────────┘
             │
┌─────────────────────────────────────────────┐
│           COMPUTE WHOLE TIME PERIOD          │  ～S12
└─────────────────────────────────────────────┘
             │
┌─────────────────────────────────────────────┐
│   CREATE PLAN OF EXPANSION PASSAGE PROCESS   │  ～S14
│          AND FOLLOWING CULTURE PROCESS       │
└─────────────────────────────────────────────┘
             │
┌─────────────────────────────────────────────┐
│      CREATE PLAN OF THAWING PROCESS,         │
│   MAINTENANCE PASSAGE PROCESSES, AND         │  ～S16
│        FOLLOWING  CULTURE PROCESS            │
└─────────────────────────────────────────────┘
             │
┌─────────────────────────────────────────────┐
│      DISPLAY AND CONFIRM CELL CULTURE PLAN   │  ～S18
└─────────────────────────────────────────────┘
             │
┌─────────────────────────────────────────────┐
│   CREATE EXPERIMENT DEFINITION DATA FOR      │
│ EACH PROCESSING AND STORE IN EXPERIMENT      │  ～S310
│            DEFINITION DB                     │
└─────────────────────────────────────────────┘
             │
┌─────────────────────────────────────────────┐
│   SET EXPERIMENT DEFINITION DB ACCESSIBLE BY │  ～S312
│        WORK INSTRUCTION DEVICE               │
└─────────────────────────────────────────────┘
             │
┌─────────────────────────────────────────────┐
│ NOTIFY WORK INSTRUCTION DEVICE OF EXPERIMENT │  ～S314
│   DEFINITION DATA CREATION COMPLETION        │
└─────────────────────────────────────────────┘
             │
       ┌───────────┐
       │    END    │
       └───────────┘
```

## FIG.30A

20

**WORKBENCH ROBOT**

LOCAL CONTROLLER ～21

INTEGRATION CONTROLLER ～30

20

**WORKBENCH ROBOT**

LOCAL CONTROLLER ～21

10

**MOBILE MANIPULATOR**

LOCAL CONTROLLER ～11

10

**MOBILE MANIPULATOR**

LOCAL CONTROLLER ～11

## FIG.30B

20

**WORKBENCH ROBOT**

LOCAL CONTROLLER ～21

20

**WORKBENCH ROBOT**

LOCAL CONTROLLER ～21

10

**MOBILE MANIPULATOR**

LOCAL CONTROLLER ～11

INTEGRATION CONTROLLER ～30

10

**MOBILE MANIPULATOR**

LOCAL CONTROLLER ～11

## FIG.31A

| | |
|---|---|
| **WORKBENCH ROBOT** — 20<br>LOCAL CONTROLLER — 21<br>DISTRIBUTED CONTROLLER — 70 | **WORKBENCH ROBOT** — 20<br>LOCAL CONTROLLER — 21<br>DISTRIBUTED CONTROLLER — 70 |
| **MOBILE MANIPULATOR** — 10<br>LOCAL CONTROLLER — 11<br>DISTRIBUTED CONTROLLER — 70 | **MOBILE MANIPULATOR** — 10<br>LOCAL CONTROLLER — 11<br>DISTRIBUTED CONTROLLER — 70 |

## FIG.31B

| | |
|---|---|
| **WORKBENCH ROBOT** — 20<br>LOCAL CONTROLLER — 21<br>DISTRIBUTED CONTROLLER — 70 | **WORKBENCH ROBOT** — 20<br>LOCAL CONTROLLER — 21<br>DISTRIBUTED CONTROLLER — 70 |

DISTRIBUTED CONTROLLER — 70

| | |
|---|---|
| **MOBILE MANIPULATOR** — 10<br>LOCAL CONTROLLER — 11<br>DISTRIBUTED CONTROLLER — 70 | **MOBILE MANIPULATOR** — 10<br>LOCAL CONTROLLER — 11<br>DISTRIBUTED CONTROLLER — 70 |

## INTERNATIONAL SEARCH REPORT

| International application No. |
| --- |
| **PCT/JP2023/040475** |

| A. | CLASSIFICATION OF SUBJECT MATTER |
| --- | --- |

*C12M 1/00*(2006.01)i
FI:   C12M1/00 C

According to International Patent Classification (IPC) or to both national classification and IPC

| B. | FIELDS SEARCHED |
| --- | --- |

Minimum documentation searched (classification system followed by classification symbols)

C12M1/00

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

JSTPlus/JMEDPlus/JST7580 (JDreamIII); CAplus/MEDLINE/EMBASE/BIOSIS (STN)

| C. | DOCUMENTS CONSIDERED TO BE RELEVANT |
| --- | --- |

| Category* | Citation of document, with indication, where appropriate, of the relevant passages | Relevant to claim No. |
| --- | --- | --- |
| X | WO 2019/069378 A1 (OLYMPUS CORPORATION) 11 April 2019 (2019-04-11) abstract, paragraphs [0011], [0024] | 1-3 |
| Y | abstract, paragraphs [0011], [0024] | 7 |
| A | | 4-6 |
| Y | 夏目 徹, ヒト型汎用ロボットによるライフサイエンスの近代化, 日本ロボット学会誌, 2013, vol. 31, no. 4, pp. 376-379, (NATSUME, Tohru. A Novel Humanoid Robotics Platform, Compatible with Standard Lab Equipment, to Increase Productivity and Data Quality. Journal of the Robotics Society of Japan.) fig. 1, 2 | 7 |
| A | | 1-6 |
| A | JP 2005-224106 A (HITACHI MEDICAL CORP.) 25 August 2005 (2005-08-25) claims | 1-7 |

| ☐ Further documents are listed in the continuation of Box C. | ☑ See patent family annex. |
| --- | --- |

| * Special categories of cited documents: | "T" later document published after the international filing date or priority date and not in conflict with the application but cited to understand the principle or theory underlying the invention |
| --- | --- |
| "A" document defining the general state of the art which is not considered to be of particular relevance | |
| "D" document cited by the applicant in the international application | "X" document of particular relevance; the claimed invention cannot be considered novel or cannot be considered to involve an inventive step when the document is taken alone |
| "E" earlier application or patent but published on or after the international filing date | |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| **22 January 2024** | **30 January 2024** |

| Name and mailing address of the ISA/JP | Authorized officer |
| --- | --- |
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (July 2022)

**INTERNATIONAL SEARCH REPORT**
Information on patent family members

International application No.

**PCT/JP2023/040475**

| Patent document cited in search report | | | Publication date (day/month/year) | Patent family member(s) | Publication date (day/month/year) |
|---|---|---|---|---|---|
| WO | 2019/069378 | A1 | 11 April 2019 | US 2020/0234032 A1 A1<br>abstract, paragraphs [0022], [0061], [0062] | |
| JP | 2005-224106 | A | 25 August 2005 | (Family: none) | |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

• JP 2004290147 A